# EUROPEAN PATENT SPECIFICATION

(11) **EP 4 388 318 B1**
(45) Date of publication and mention of the grant of the patent: **30.09.2026**
(21) Application number: 22765244.3
(22) Date of filing: 09.08.2022
(51) Int. Cl.: G01N 33/557, A61B 5/00

(54) **METHOD AND SYSTEM FOR THE MONITORING OF AN ANALYTE OF INTEREST**
VERFAHREN UND SYSTEM ZUR ÜBERWACHUNG EINES BESTIMMTEN ANALYTEN
PROCÉDÉ ET SYSTÈME POUR LA SURVEILLANCE D'UN ANALYTE D'INTÉRÊT

(30) Priority: 17.08.2021 NL 2028978
(43) Date of publication of application: 26.06.2024
(73) Proprietor: Technische Universiteit Eindhoven, 5612 AE Eindhoven (NL)
(72) Inventor: PRINS, Menno Willem José, 5612 AE EINDHOVEN (NL); LUBKEN, Rafiq Milan, 5612 AE EINDHOVEN (NL)
(74) Representative: Algemeen Octrooi- en Merkenbureau B.V.
(86) International application number: PCT/NL2022/050462
(87) International publication number: WO 2023/022589

(56) References cited:
- WO-A1-2018/158469
- LIN YU-TING ET AL: "Click-Coupling to Electrostatically Grafted Polymers Greatly Improves the Stability of a Continuous Monitoring Sensor with Single-Molecule Resolution", ACS SENSORS, vol. 6, no. 5, 28 May 2021 (2021-05-28), US, pages 1980 - 1986, XP055967510, ISSN: 2379-3694, Retrieved from the Internet <URL:https://www.ncbi.nlm.nih.gov/pmc/articles/PMC8165697/pdf/se1c00564.pdf> DOI: 10.1021/acssensors.1c00564
- YAN JUNHONG ET AL: "Continuous Small-Molecule Monitoring with a Digital Single-Particle Switch", ACS SENSORS, vol. 5, no. 4, 24 April 2020 (2020-04-24), US, pages 1168 - 1176, XP055967513, ISSN: 2379-3694, Retrieved from the Internet <URL:https://www.ncbi.nlm.nih.gov/pmc/articles/PMC8177406/pdf/se0c00220.pdf> DOI: 10.1021/acssensors.0c00220
- VISSER EMIEL W. A. ET AL: "Continuous biomarker monitoring by particle mobility sensing with single molecule resolution", NATURE COMMUNICATIONS, vol. 9, no. 1, 29 June 2018 (2018-06-29), XP055794597, Retrieved from the Internet <URL:http://www.nature.com/articles/s41467-018-04802-8> DOI: 10.1038/s41467-018-04802-8
- LUBKEN RAFIQ M. ET AL: "Sensing Methodology for the Rapid Monitoring of Biomolecules at Low Concentrations over Long Time Spans", ACS SENSORS, vol. 6, no. 12, 2 December 2021 (2021-12-02), US, pages 4471 - 4481, XP055967501, ISSN: 2379-3694, Retrieved from the Internet <URL:https://pubs.acs.org/doi/pdf/10.1021/acssensors.1c01991> DOI: 10.1021/acssensors.1c01991

## Description

### Technical field

The present invention relates to a method and a system for the continuous monitoring or intermittent testing of an analyte of interest, such as a chemical, biochemical, or biological substance or structure, present in or at a system of interest, such as a container, a reservoir, a reactor, a tube, a line, a vessel, a lumen, a tissue, an organ, or an organism.

### Background

Biological systems and biotechnological processes exhibit time-dependencies that are at the most basic level controlled by the dynamics of the constituting analytes, such as small molecules, hormones, proteins, and nucleic acids. This calls for measurement technologies that allow the monitoring of analyte concentrations, for instance in order to serve fundamental research on biological and biomedical dynamics, to enable the development of patient monitoring strategies based on real time biomolecular data, as well as to enable the development of closed loop control strategies in biotechnological applications. Desirable characteristics of a generic monitoring technology are (1) sensitive and specific measurements, (2) small time delays between sampling input and data output, (3) short time intervals between successive measurements, and (4) a long total time span over which time-dependent analyte concentration data can be recorded.

It is a fundamental challenge to develop a technology that can rapidly monitor low-concentration analytes over long time spans. Sensitive measurement technologies are available, such as ELISA and flow cytometry, but these methods consume reagents for every sample taken and every concentration determined, which complicates applications where analyte concentrations need to be monitored over long time spans. On the other hand, sensing technologies that can operate without consuming reagents, such as surface plasmon resonance, redox cycling and quartz crystal microbalance, have not been designed for monitoring analytes at low concentrations, such as in the picomolar and sub-picomolar range.

A generic bioanalytical principle used to quantify analyte concentrations with high specificity and sensitivity, is the biochemical affinity of specific binding sites. The binding sites can be effectuated by binding materials (such as molecularly imprinted polymers or other nanomaterials) or by binder molecules (such as antibodies, aptamers, proteins, nucleic acids, and the like). Typically, binding sites are effectuated by binder molecules, with at least one binding site per binder molecule for binding to the analyte, where the binder molecules are mobile or immobilized. The specificity originates from molecular interactions such as charge, hydrogen bonding, van der Waals forces, and hydrophobic and steric effects. To be able to measure analytes at low concentrations with high sensitivity, binder molecules are needed that have strong interactions with their target analytes, which corresponds to high binding energies, low equilibrium dissociation constants *K_{d}*, and low dissociation rate constants *k*_{off}. However, this conflicts with the desire to have small time delays, because low dissociation rate constants would imply a need for long incubation times to reach equilibrium. Furthermore, low dissociation rate constants result in a slow reversibility, which conflicts with the wish to enable short time intervals between successive measurements.

International patent application published under number WO 2018/158469 A1 discloses a biosensing device for continuous monitoring of an analyte in a fluid matrix including an electromagnetic excitation element, a biosensing surface, an opposing surface, a separation component, light collection optics, a light sensor, an image recording and analysis system, and an excitation control system. The separation component of the biosensing device is connected to the biosensing surface and to the opposing surface, forming a concave gap region between the biosensing surface and the opposing surface. The biosensing surface comprises biosensing particles sensitive to electromagnetic signals from the electromagnetic excitation element, where an optical response of the biosensing particles to the electromagnetic signals is adapted to change in the presence of an analyte in the gap region. The light collection optics couple light emitted from the biosensing particles on the biosensing surface to the light sensor. The image recording and analysis system is connected to the light sensor and processes light signals from the biosensing particles to determine presence or absence or concentration of the analyte in the fluid matrix.

### Description of the invention

The present invention relates to a method and a biosensing system that enables rapid monitoring of low concentrations of analytes. The method is based on the use of binder molecules with a high affinity in a limited-volume assay, with a reversible detection principle and time-controlled sampling of the analyte of interest. The system allows optimal tradeoffs between time characteristics and sensitivity. The present invention presents the measurement concept, time-dependencies of sensor signals, and a comprehensive analysis of the achievable time characteristics and sensitivity as a function of sensor design parameters. It was found that the sensing methodology enables precise and accurate quantification of low analyte concentrations, with time delays and interval times that are much shorter than the time dictated by the dissociation rate constant of the binder molecules. Furthermore, due to the reversible detection method, measurements can in principle be done over an endless time span.

In order to provide such a rapid monitoring of low concentrations of analytes, the present invention provides hereto a method for the continuous monitoring or intermittent testing of an analyte of interest, such as a chemical, biochemical, or biological substance or structure, present in or at a system of interest, such as a container, a reservoir, a reactor, a tube, a line, a vessel, a lumen, a tissue, an organ, or an organism, wherein a fluid or another viscoelastic medium or material comprises the analyte of interest, by measuring the concentration of the analyte of interest in a measurement chamber, wherein the measurement chamber comprises an effective number of binding sites (*N_{b}*), wherein the binding sites have a binding affinity to the analyte of interest, wherein the measurement chamber has an effective volume (*V_{ch}*) in which the analyte of interest has a significant probability to encounter the binding sites, and wherein the method comprises the step of providing a time-dependent sampling of the analyte of interest, by providing a time-dependent exchange of analyte between the system of interest and the effective volume (*V_{ch}*) of the measurement chamber, by performing at least one exchange modulation cycle comprising the following successive steps:
a) facilitating a primary exchange phase having a characteristic time of primary exchange (*τ*_{*pr.exch*.}) and a duration of primary exchange (*t_{pr.exch.}*);
b) facilitating a primary-to-secondary switching phase having a characteristic primary-to-secondary switching time (*τ_{pr.sec.switch}*) and a primary-to-secondary switching duration (*t_{pr.sec.switch}*); and
c) facilitating a secondary exchange phase having a characteristic time of secondary exchange (*τ_{sec.exch}.*) and a duration of secondary exchange (*t_{sec.exch}.*),
wherein the exchange modulation cycle is repeated for any time-dependent sampling further provided, and wherein:
- the number of binding sites (*N_{b}*) and/or the effective volume (*V_{ch}*) of the measurement chamber is selected such that the effective volumetric binding site concentration (*C_{b,ch}*) in the measurement chamber is present in excess compared to the effective equilibrium dissociation constant (*K_{d}*) of the affinity binding between analyte of interest and binding sites, where *C_{b,ch}* is expressed as *N_{b}*/*V_{ch}*;
- the sum of the duration of primary exchange (*t_{pr.exch.}*) and the primary-to-secondary switching duration (*t_{pr.sec.switch}*) and the duration of secondary exchange (*t_{sec.exch}.*) is larger than a characteristic time-to-equilibrium (*τ*) in the measurement chamber;
- the concentration of the analyte of interest is determined by direct or indirect measuring the time-development of the amount of analyte of interest bound to at least one or more binding sites; and
- the direct or indirect measuring of the time-development of the amount of analyte of interest bound to at least one or more binding sites involves at least two measurements performed at different time-points in at least one exchange modulation cycle.

It is noted that the terms "direct measuring" or "indirect measuring" in relation to the amount of analyte of interest bound to at least one or more binding sites relate, respectively, to the direct measuring of the analyte of interest or the indirect measurement of the analyte of interest by measuring an analyte-analogue or analyte derivative or another molecule or another substance or another object or a chemical or physical property related to the analyte.

In the method of the present invention, the binding sites may be present on or in a supporting structure, such as a planar surface, a surface with concave or convex structure, a chemically and/or physically patterned surface, a particle, a polymer, or a porous matrix. Alternatively or in addition to the supporting structure the binding sites may be present in said fluid or another viscoelastic medium or material comprising the analyte of interest.

Further to the method defined above, the exchange modulation cycle may be repeated by performing an additional step d) after step c) comprising:
d) facilitating a secondary-to-primary switching phase having a characteristic secondary-to-primary switching time (*τ_{sec.pr.switch}*) and a secondary-to-first switching duration (*t_{sec.pr.switch}*), and wherein:
- the sum of the duration of primary exchange *(t_{pr.exch.})* and the primary-to-secondary switching duration (*t_{pr.sec.switch}*) and the duration of secondary exchange (*t_{sec.exch}.*) and the secondary-to-primary switching duration (*t_{sec.pr.switch}*) is larger than a characteristic time-to-equilibrium (*τ*) in the measurement chamber.

As used herein, the terms "characteristic time of primary exchange" and "characteristic time of secondary exchange" refer to the time required to achieve 63% analyte exchange in the measurement chamber, i.e. the time required to evolve from a starting condition to a condition where a significant amount (63%) has been achieved of the change of concentration due to analyte exchange. Also, as used herein, the terms "characteristic primary-to-secondary switching time" and "characteristic secondary-to-primary switching time" refer to the time required to achieve 63% switching from primary to secondary phase and secondary to primary phase, respectively.

The term "binding sites" as used herein refers to "binders", "binder molecules" or "binder materials", which are able to bind and to form "analyte-binder complexes" with the analyte of interest.

The analyte of interest may be a chemical, a biochemical, or a biological substance or structure. The analyte of interest may be a supramolecular analyte, e.g. a virus particle, a supramolecular structure, a cell fragment, an intracellular body, an extracellular vesicle, a nanoparticle.

The time-dependent sampling of the analyte of interest according to the method of the present invention may be effectuated by time-dependent exchange of analyte by diffusion, advection, or by another active or passive physicochemical analyte transport method, or by a combination thereof.

In an embodiment of the method of the present invention, the duration of primary exchange (*t_{pr.exch.}*) may be smaller than the characteristic time-to-equilibrium (*τ*) in the measurement chamber. Alternatively or additionally, the duration of primary exchange (*t_{pr.exch.}*) may be larger than the characteristic time of primary exchange (*τ_{pr.exch.}*). The duration of primary exchange (*t_{pr.exch.}*) is preferably larger than one-hundredth of the characteristic time of primary exchange (*τ_{pr.exch.}*).

In a further embodiment of the method of the present invention, the primary-to-secondary switching duration (*t_{pr.sec.switch}*) may be larger than the characteristic primary-to-secondary switching time (*τ_{pr.sec.switch}*), and/or the characteristic primary-to-secondary switching time (*τ_{pr.sec.switch}*) may be smaller than the characteristic time-to-equilibrium (*τ*) in the measurement chamber.

Also, in the case of step d), the secondary-to-primary switching duration (*t_{sec.pr.switch}*) may be larger than the characteristic secondary-to-primary switching time (*τ_{sec.pr.switch}*), and/or the characteristic secondary-to-primary switching time (*τ_{sec.pr.switch}*) may be smaller than the characteristic time-to-equilibrium (*τ*) in the measurement chamber.

In an embodiment of the method of the present invention, the sum of the duration of primary exchange (*t_{pr.exch.}*) and the primary-to-secondary switching duration (*t_{pr.sec.switch}*) may be smaller than the characteristic time-to-equilibrium (*τ*) in the measurement chamber.

In an embodiment of the method of the present invention, the duration of the secondary exchange (*t_{sec.exch}.*) may be smaller than the characteristic time of secondary exchange (*τ_{sec.exch.}*).

The at least one exchange modulation cycle may comprises two or more exchange modulation cycles, preferably at least three, four, five, six, seven, eight, nine, ten exchange modulation cycles.

In an embodiment, the at least one exchange modulation cycle comprises two or more exchange modulation cycles and wherein the measuring of the time-development of the amount of analyte of interest bound to at least one or more binding sites involves at least two measurements performed at different time-points in at least one exchange modulation cycle.

It is further noted that the facilitating of the phases during the at least one exchange modulation cycle may be performed by diffusion, advection, or by another active or passive physicochemical analyte transport method, or by a combination thereof. The phases of the at least one exchange modulation cycle may be effectuated by controlling the transport method in time.

It was further found that the increase or decrease of the time-development of the amount of analyte of interest bound to at least one or more binding sites during an exchange modulation cycle may depend on the amount of analyte of interest bound to at least one or more binding sites in said exchange modulation cycle and in a previous exchange modulation cycle.

In a further embodiment, the binding of the analyte of interest to a binding site is measured by:
- a property of the analyte of interest, such as by charge, refractive index, fluorescence, luminescence, absorption, change of conformation, enzymatic activity, colour, or mass; or
- a signal from another object, such as a molecule, substance, particle, label, surface, or a combination thereof, for example by energy transfer, resonance, scattering, absorption, motion, charge, refractive index, fluorescence, luminescence, change of conformation, enzymatic activity, colour, or mass,
wherein the measurement involves binding, conversion, competition, inhibition, displacement, amplification, molecular cascade, or sandwich formation, or a combination thereof.

The present invention further relates to a system for continuously monitoring or intermittently testing at least one analyte of interest, wherein the system comprises:
- a measurement chamber comprising a number of binding sites (*N_{b}*), wherein the binding sites are able to bind the analyte of interest, and wherein the measurement chamber has an effective volume (*V_{ch}*);
- at least one exchange port, such as a tube, a channel, an opening, a connector, a valve, a permeable or semipermeable material, or a membrane, for time-dependent sampling of the analyte of interest involving transport into and/or out of the measurement chamber,
wherein the system is configured to perform the method according to the present invention and defined in the previous paragraphs.

The system of the present invention is preferably configured to monitor:
- one analyte of interest; or
- multiple analytes of interest, wherein the measurement chamber comprises multiple binding sites, and wherein each of the multiple binding sites is able to bind a specific analyte of interest selected from the group of multiple analytes of interest to be monitored.

In an embodiment of the present invention the system is configured to monitor multiple analytes of interest, and wherein the system is further configured to perform multiple methods according to the present invention in parallel, wherein each of the methods performed monitors one analyte of interest of the multiple of analytes of interest to be monitored.

In another aspect of the present invention, the invention relates to a biosensor device according to the present invention for use in *in vivo* biosensing, *ex vivo* biosensing, or *in vitro* biosensing, such as in, but not limited to, *in vitro* diagnostic testing, personal monitoring, animal testing, point-of-care testing, medical applications, life science applications, pharmaceutical applications, environmental testing, food testing, process monitoring, process control, water monitoring, environmental monitoring, air quality monitoring, vapor testing, breath fluid testing, chemical monitoring, forensics, biological, biomedical, or pharmaceutical research, agriculture, or to monitor assays with live cells, tissue, or an organ, organ-on-a-chip, or for measurement-and-control, closed loop control, real-time monitoring, and early warning applications.

The biosensor of the present invention may be used for continuous monitoring or for intermittent testing.

The analyte of interest may be measured continuously, i.e. by continuously taking samples for measuring, or non-continuously, i.e. by taking discrete samples for measuring.

The biosensor of the present invention may be prepared for immediate use, or rapid use, or plug-and-play use.

The biosensor may include transport methods such as diffusion, advection, acoustic excitation, magnetic actuation, thermal transport, convection, electrophoresis, optical excitation, syringe pumping, peristaltic pumping, membrane pumping, centrifugal excitation, actuation based on a fluid-fluid meniscus, actuation based on bubbles or droplets, ultrasonic excitation, actuation by electric fields, and the like.

The biosensor may be suited for multiplexing, i.e. measurement of several different analytes simultaneously or in parallel.

The biosensor may be used with a variety of binders, e.g. molecules, molecular constructs, and materials, such as, but not limited to, oligonucleotides, proteins, peptides, polymers, aptamers, small molecules, sugars, and molecularly imprinted polymers

The analyte of interest may be a chemical, biochemical, or biological substance or structure. The analyte of interest may be measured directly in the system of interest, e.g. the measurement system may be provided in a flow path of the medium in the system of interest. Alternatively, a serial process may be applied, e.g. the medium may be sampled from the system of interest and may be transported to another system of interest, for example, the system of interest may be an organism, an organ, a tissue, a vessel, a cell system, a unit operation, a reactor, a lumen, a line, a tube, a bag, a receptacle, a chip, a well plate, an intermediate container, a reservoir, a chamber, a drip chamber.

The medium or sample may be pretreated in a sampling system, e.g. filtration, dilution, reagent addition, splitting, grinding, mixing, temperature treatment, heating, cooling, illumination, acoustic excitation, centrifugation, pressure application, under- or overpressure, cell lysis, enzymatic treatment, radiation treatment, amplification, separation, concentration, extraction, degassing, exposure to gas, removal of gas, and the like.

The biosensor may be provided with a functionality to provide wash steps, to add or release molecules or materials, to elute molecules or materials, or reset, regenerate or (re)activate the sensor or parts thereof.

### Further embodiments of the invention

In further embodiments of the device or method of the present invention, the biosensor may not be in direct contact with a system of interest, or may be in direct contact with a system of interest. Alternatively, the biosensor may be embedded or integrated or implanted in a system of interest. The biosensor can be placed at a distance from the system of interest. However, the biosensor may be located near the system of interest, on the system, wirelessly integrated, or the like. Samples can be put in a container and then transported to the biosensing system (sometimes called at-line or offline operation), samples can be taken and automatically transported to the biosensing system (sometimes called on-line operation), or the biosensing system can be fully integrated with the system of interest (sometimes called in-line operation).

In further embodiments of the present invention, the device or method may be connected to or integrated in an industrial system or process, a fermenter, a bioreactor, an on-body device, a catheter, an in-body device, a wearable device, or an insertable device.

In a biosensing system with monitoring functionality, time-dependent samples can be taken, measurement data may be recorded, and a time profile may be established of analyte concentration. Also, a biosensor may be configured to receive a series of samples (from the same or from different sources) where the series of samples are serially or parallelly measured on the biosensor and result in time-dependent data that relate to different samples that have been supplied to the biosensor.

In further embodiments of the present invention, the device or method may be combined with a method or device module for sample pre-treatment or analyte pre-treatment, e.g. reagent addition, dilution, filtration, extraction, enrichment, purification, separation, amplification, change of buffer condition, stabilization, (dis)aggregation, or removal, modification, or addition of a chemical group or a biochemical domain or residue or moiety.

In further embodiments of the present invention, the device or method may be combined with a method or device module for optimization or control of operation, e.g. temperature, humidity, pressure, light conditions, vibration conditions, sound conditions, sterility, hygiene, ingress protection, cleaning, parts replacement, easy maintenance, calibration, and the like.

### Detailed description of the invention

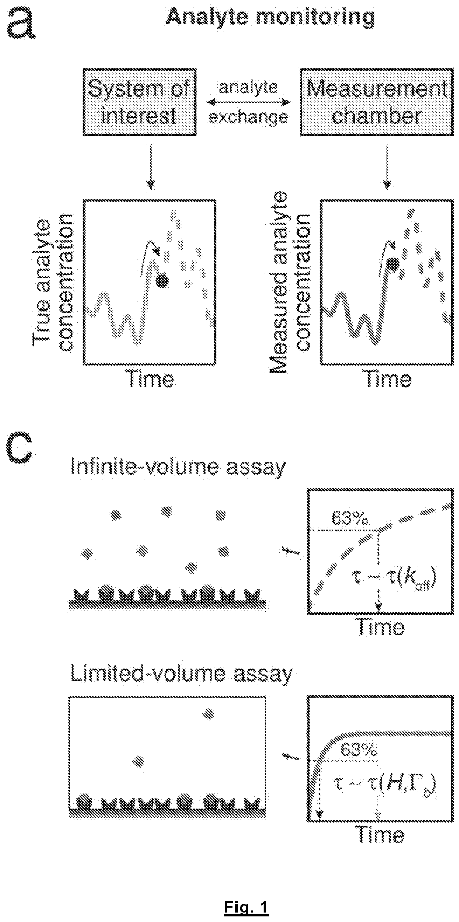

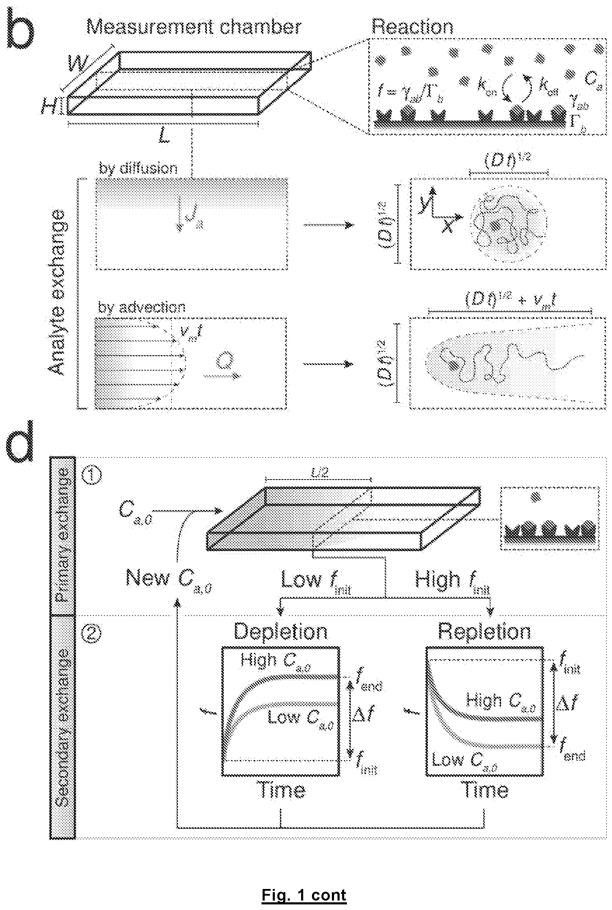

The basic concepts of the sensing methodology of the invention are sketched and exemplified in Figure 1. The sensing system features time-dependent sampling of the analyte of interest, provided by a time-controlled analyte exchange between a biological or biotechnological system of interest and a measurement chamber (Figure 1A). The measurement chamber contains specific binder molecules from which signals are recorded. Measurement time series are recorded and translated into concentration-time profiles, which should resemble as close as possible the true concentration-time profile of analytes in the system of interest. During the exchange of analytes, various processes occur, such as mass transport by advection and diffusion, and association and dissociation of analytes to binder molecules (Figure 1B). A rectangular measurement chamber is assumed with height *H,* width *W,* and length *L,* resulting in an effective volume *V_{ch}* of the measurement chamber, where *V_{ch} = HWL*. The sensor surface is provided with binder molecules, where association and dissociation of analytes occurs. The binding process between analyte and binding sites can be described by an effective equilibrium dissociation constant (K_{d}), which represents a balance between on the one hand dissociation pathways that liberate binding sites or make them available, and on the other hand association pathways that occupy or block binding sites. To illustrate the concept of the invention, a simple bi-molecular system is assumed with reversible one-to-one binding between analyte (*a*) and binder (*b*): *a* + *b* 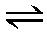 *ab*. The rates of association and dissociation depend on the association rate constant *kₒₙ,* the dissociation rate constant *k_{off},* the density Γ*_{b}* of binder molecules, and the analyte concentration *Cₐ* at the sensor surface. These processes result in a time-dependent density *γ_{ab}* of analyte-binder complexes, also represented as a fractional occupancy *f* of binder molecules occupied by analytes: *f = γ_{ab}*/Γ*_{b}*. Variables *γ_{ab}* and *f* are changing as a function of analyte concentration and time. In an affinity-based sensor, the observed signal scales with *f,* therefore *f* is used in the present invention as the sensor read out parameter to determine the analyte concentration. Analyte exchange between the system of interest and the measurement chamber is facilitated by diffusion or a combination of diffusion and advection. A net molar flux *Jₐ* (orange gradient), caused by a concentration difference between the system of interest and the measurement chamber, facilitates diffusive mass transport of analytes between the measurement chamber and the system of interest. A developed laminar flow profile with flow rate *Q* and mean flow speed *vₘ* (black arrows) facilitates advective mass transport of analytes into the measurement chamber. Here it is assumed that diffusive transport occurs in both the longitudinal (x-direction) and the lateral direction (y-direction) and scales with the diffusion coefficient *D*, while advective transport occurs only in the longitudinal direction and scales with the mean flow speed *vₘ*.

Figure 1C sketches two different sensor designs, namely an infinite-volume assay and a limited-volume assay. The graphs visualize the fractional occupancy *f* of binder molecules occupied by analytes as a function of time, with corresponding characteristic time-to-equilibrium *τ*, defined as the time needed to attain 63% of the difference between the starting level and the equilibrium level of *f* (see Supplementary Information 2). In an infinite-volume assay, continuous analyte exchange is enabled between the system of interest and the measurement chamber, where the system of interest is assumed to be much larger than the measurement chamber. The continuous analyte exchange could for example be facilitated by diffusive analyte transport across a contact area between the system of interest and the measurement chamber, while another configuration may involve a continuous flow of sample fluid, provided into the measurement chamber from the system of interest. When the analyte exchange is applied effectively and with negligible time delay, then the analyte concentration at the sensor surface (*Cₐ*) is equal to the input analyte concentration (*C*_{*a*,0}). In case of low analyte concentrations (*C*_{*a,*0} *< K_{d}*), the infinite-volume assay condition leads to a characteristic time-to-equilibrium *τ* ≅ 1/*k*_{off} (see Supplementary Information 2). This implies that the time-to-equilibrium is determined by the dissociation rate constant *k*_{off}, so this time is long when the binder molecules strongly bind to the analytes.

The sensor design with a limited-volume assay has very different properties. Here, analyte exchange is limited, so that the binder molecules in the measurement chamber interact with only a limited sample volume and therefore with a limited amount of analytes. An effective volumetric concentration of binder molecules is defined *C_{b,ch} =* Γ*_{b}*/*H = N_{b}*/ *V_{ch}*, where *N_{b}* is the number of binder molecules present in the measurement chamber. When *C_{b,ch}* is high, with *C_{b,ch} > C*_{*a,*0} and *C_{b,ch} > K_{d}*, then the time-to-equilibrium *τ* of the assay becomes dominated by the high concentration of binder molecules. When diffusional transport delays can be ignored, then the time-to-equilibrium of the assay equals *τ* ≅ 1/(*k*ₒₙ*C_{b,ch}*) (see Table 1 and Supplementary Information 1 and 2). Thus, the time-to-equilibrium of the limited-volume assay is determined by the association rate constant and the effective volumetric concentration *C_{b,ch}* of binder molecules, which leads to equilibrium timescales that are much shorter than the time-to-equilibrium of the infinite-volume assay.

In monitoring applications, one wants to record measurements with one and the same sensor over long time spans. To realize the limited-volume assay principle in a monitoring application, the sensor needs to be switched between two different conditions: an open condition and a closed condition. In the open condition, analytes are exchanged effectively between the system of interest and the measurement chamber, as sketched in Figure 1A and 1B (see also Supplementary Information 5). In the closed condition, analytes are exchanged ineffectively between the system of interest and the measurement chamber, causing a limited-volume incubation in the measurement chamber, as sketched in the bottom graph of Figure 1C. The switching concept is referred to between open and closed condition as "time-controlled analyte exchange" or as "providing a time-dependent exchange of analyte". Figure 1D illustrates the operating principle for a sensor where time-controlled exchange is realized by a modulated flow. Phase 1 is the primary exchange phase, where there is effective analyte exchange between the system of interest and the measurement chamber with duration *t_{pr.exch},* due to a short characteristic time of primary exchange *τ_{pr.exch}*, so that the starting concentration in the chamber equals *C*_{*a*,0}. Phase 2 is the secondary exchange phase, where there is ineffective analyte exchange between the system of interest and the measurement chamber with duration *t_{sec.exch},* due to a long a characteristic time of secondary exchange *τ_{sec.exch}*, so that the limited-volume assay condition is provided. During the secondary exchange phase , the analyte concentration *Cₐ* in the measurement chamber decreases over time (depletion) or increases over time (repletion), depending on the initial occupation of binder molecules *f*ᵢₙᵢₜ by analytes, since analytes are exchanged ineffectively between the system of interest and the measurement chamber. When *f*ᵢₙᵢₜ is low, the concentration of analytes in the measurement chamber decreases over time, corresponding to depletion of analyte. When *f*ᵢₙᵢₜ is high, the concentration of analytes in the chamber increases over time, corresponding to repletion of analyte. It is assumed that there is no analyte exchange during the secondary exchange phase *(i.e., τ_{sec.exch}* → ∞) and that the time-to-equilibrium is not influenced by the primary exchange phase (*i.e*., *t_{pr.exch} < τ*, see Supplementary Information 6). Therefore, for known *f*ᵢₙᵢₜ, the supplied analyte concentration *C*_{*a,*0} can be derived from the measured time dependence of the fractional occupation *f*(*t*) during the secondary exchange phase. At least two measurements need to be done to determine the input analyte concentration *C*_{*a*,0}, for example a measurement at the initial value *f*ᵢₙᵢₜ and a measurement at the final value *f*_{end}, as indicated in the graph. The time required to change from the primary to the secondary exchange phase, *i.e.,* the primary-to-secondary switching phase, and the time required to change from the secondary back to the primary exchange phase, *i.e.,* the secondary-to-primary switching phase, have a characteristic switching time *τ_{switch}* and a switching duration of *t_{switch}*. Here it is assumed that *τ_{switch}* and *t_{switch}* for both switching phases are negligibly small compared to the other time scales in the monitoring system.

By sequentially applying cycles with open condition and closed condition, discrete samples with a limited volume are serially measured and result in time-dependent data that relate to the different samples supplied to the sensor. The limited-volume condition ensures that *C_{b,ch} > C*_{*a,*0} and causes the binder molecules to influence the analyte concentration *Cₐ* in the measurement chamber. Each former measurement causes a varying nonzero initial fractional occupancy *f*ᵢₙᵢₜ in the next measurement. The values of *f*ᵢₙᵢₜ and *C*_{*a,*0} determine whether depletion or repletion occurs during the incubation phase. In case of depletion, a higher input analyte concentration *C*_{*a,*0} yields a larger, positive change of fractional occupancy Δ*f* = *f*_{end} - *f*ᵢₙᵢₜ since more analytes are captured from solution, while for repletion a higher *C*_{*a,*0} yields a smaller, negative change of fractional occupancy Δ*f* since less analytes are repleted from the sensor surface into solution. An important property of the sensor is that the interactions between binder and analytes are reversible. This gives the advantage that the limited-volume assay with time-controlled analyte exchange can be used over an endless time span.

It was further found that sensor design parameters influence the time characteristics and sensitivity of the sensing methodology. The time characteristics are quantified by finite-element simulations of mass transport in the sensor and reaction processes at the sensor surface, and the sensitivity is quantified by calculating the stochastic variabilities in the measurements. The simulations and calculations are verified by experiments using a sensing technique with single-molecule resolution, called Biosensing by Particle Mobility (BPM, see Supplementary Information 7).

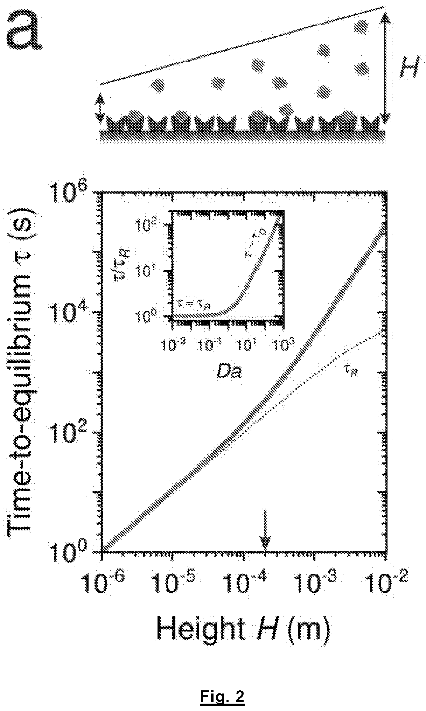

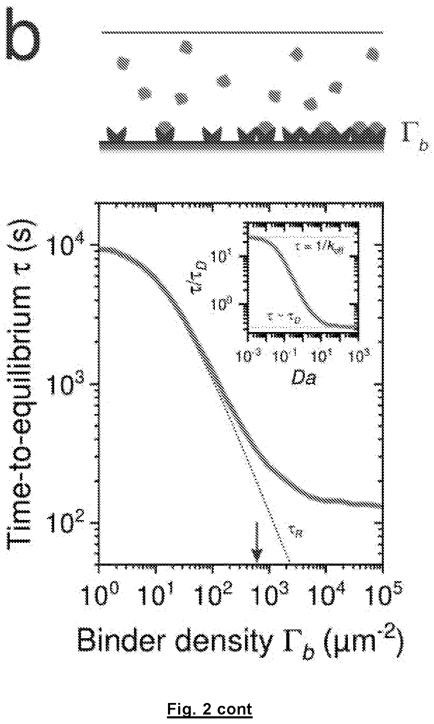

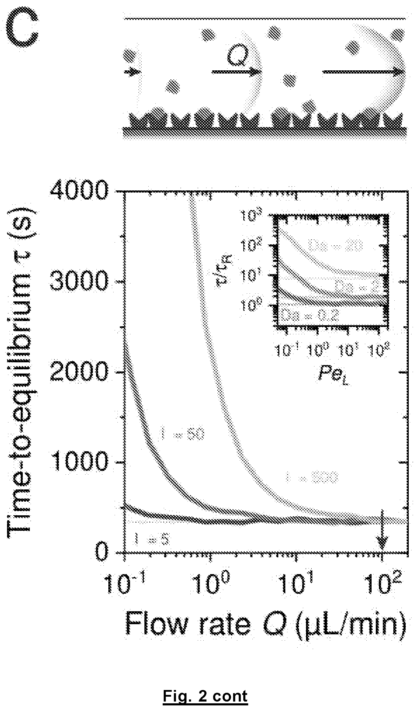

Figure 2 shows simulation results of the time-to-equilibrium of the limited-volume assay, for sensor designs with different chamber heights, different binder densities, and different flow rates, assuming standard parameter values as listed in Table 1. Figure 2A shows how the time-to-equilibrium *τ* depends on the chamber height H, for a sensor with instantaneous analyte exchange (*i.e., τ_{pr.exch}* → 0, see Supplementary Information 6 for the influence of analyte exchange on the sensor performance). The arrow on the x-axis indicates the height as listed in Table 1. The data show that the time-to-equilibrium increases with the chamber height. At small *H*, this increase in the time-to-equilibrium is caused by a decrease of the effective volumetric binder concentration *C_{b,ch} =* Γ*_{b}*/*H*, while at large *H*, this increase is caused by diffusive transport limitations. The inset shows the same data, plotted as a function of the Damköhler number (*Da = τ_{D}*/*τ_{R,LV} = kₒₙ*Γ*_{b}H*/*D*); low *Da* means that the kinetics are limited by the reaction, high *Da* means that the kinetics are limited by diffusion. To achieve a fast time-to-equilibrium, the sensor should be designed with a large *C_{b,ch}*, so a small *H.*

Figure 2B shows how the time-to-equilibrium depends on the binder density Γ*_{b}*, for a sensor with instantaneous analyte exchange. The arrow indicates the density as listed in Table 1. For small Γ*_{b}*, the time-to-equilibrium is long and determined by the dissociation rate constant (*τ* ≅ 1/*k*_{off}). For Γ*_{b} > HK_{d}* ≅ 20 µm⁻², the time-to-equilibrium decreases, until it stabilizes due to diffusive transport limitations (*τ* ≅ *τ_{D} = H²*/*D*). The inset shows the same data plotted as a function of *Da.* To achieve a fast time-to-equilibrium, the sensor should be designed with a large *C_{b,ch}*, so a large Γ*_{b}*.

Figure 2C shows how analyte exchange by advection contributes to the time-to-equilibrium per measurement cycle. The primary exchange phase involves a temporary flow of fluid into the measurement chamber, with flow rate Q and duration *t_{pr.exch}* (see Supplementary Information 6 for the influence of analyte exchange on the sensor performance). In the simulations, *t_{pr.exch}* was chosen to be equal to the characteristic time of primary exchange *τ_{pr.exch}* which equals the characteristic advection time *τ_{A}*, so *t_{pr.exch} = τ_{pr.exch} = τ_{A} = HLW*/*Q*, which means that a total fluid volume is displaced equal to the volume *V_{ch}* of the measurement chamber. The time-to-equilibrium *τ*, which now includes a contribution *t_{pr.exch}* related to the exchange, is shown as a function of flow rate, for several values of the chamber aspect ratio *λ = L*/*H*. The arrow indicates the flow rate as listed in Table 1. For small *Q* the observed *τ* is limited by *t_{pr.exch}, i.e.,* the advective transport of analytes from the inlet toward the point of sensing at a distance *L*/2 from the inlet, as sketched in Figure 1D. For increasing *λ*, *i.e*., increasing *L* with a fixed *H,* the time-to-equilibrium increases since *τ_{A}* (and thus also *t_{pr.exch}*) increases. For increasing *Q*, the time-to-equilibrium decreases, until it stabilizes at a level where the reaction and diffusion times determine the observed *τ*. The inset shows the same data (*Da =* 2), supplemented with *Da =* 0.2 (reaction-limited) and Da = 20 (diffusion-limited), plotted as a function of the longitudinal Péclet number ( ${Pe}_{L} = {τ}_{D} / {τ}_{A} = \frac{Q}{λDW}$); low *Pe_{L}* means that the analyte exchange is limited by advection, high *Pe_{L}* means that the analyte exchange is limited by diffusion. A low *Pe_{L}* causes a long time-to-equilibrium due to slow mass transport by advection. Increasing *Pe_{L}* results in a decrease of the time-to-equilibrium due to rapid filling of the chamber, until it stabilizes at a *τ* value equal to the value indicated in Figure 2A. Figure 2C shows the flow rate required to minimize the influence of the primary exchange phase on the time-to-equilibrium. In the following sections, exchange with a high *Pe_{L}* is assumed, *i.e.,* rapid filling of the measurement chamber, causing the time-to-equilibrium to be independent of the primary exchange phase.

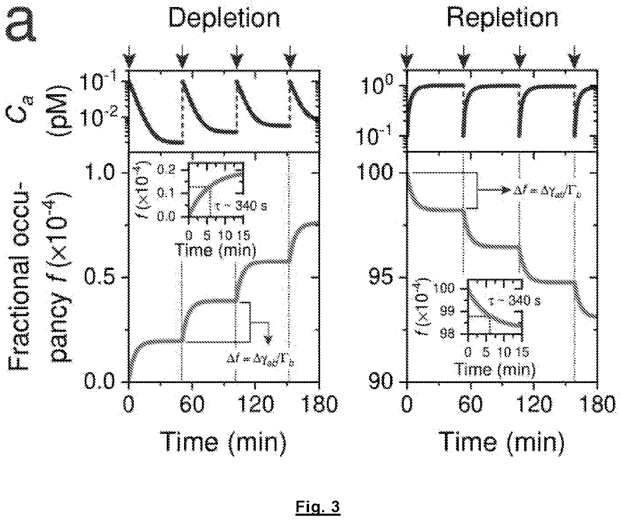

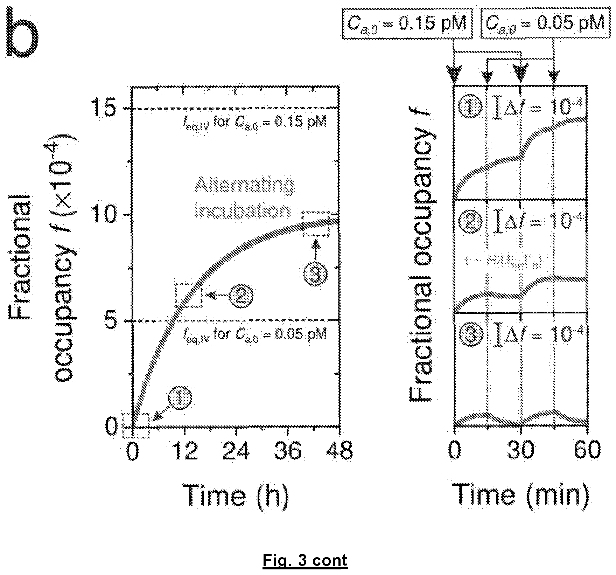

Figure 3 shows simulation results for a limited-volume assay with time-controlled analyte exchange. The analyte exchange is assumed to be instantaneous and the secondary exchange phase includes mass transport by diffusion and reaction kinetics within the measurement chamber itself, but no analyte exchange between the system of interest and the measurement chamber. Figure 3A shows data for repeated incubations with *C*_{*a,*0} = 0.1 pM. The analyte concentration *Cₐ* in the measurement chamber (brown line) and the fractional occupancy *f* of the binders by analytes (orange line) are plotted as a function of time, for conditions of analyte depletion (left) and analyte repletion (right). The time-to-equilibrium *τ* of each incubation equals approximately 340 s (see Figure 2), having contributions from reaction (*τ_{R}* = 200 s) and diffusion (*τ_{D}* = 400 s). The contribution from the reaction to the time-to-equilibrium is much smaller than 1/*k*_{off} = 10⁴ s, the value that would have been observed in case of an infinite-volume assay (cf. Figure 1C). In absence of diffusion limitations, the acceleration that can be achieved with a limited-volume assay compared to an infinite-volume assay equals $α = \frac{1 / {k}_{off}}{{τ}_{R , LV}} = \frac{{k}_{on} {Γ}_{b}}{H {k}_{off}} = {C}_{b , ch} / {K}_{d}$ , which clarifies how the speed of the assay is directly related to the ratio between effective volumetric binder concentration and the equilibrium dissociation constant.

Figure 3B shows the response of a limited-volume assay with time-controlled analyte exchange where the sensor is incubated with an alternating analyte concentration (orange line): an analyte concentration below 0.1 pM (*C*_{*a,*0} = 0.05 pM) and an analyte concentration above 0.1 pM (*C*_{*a,*0} = 0.15 pM). The infinite-volume equilibrium fractional occupancy *f*_{eq,IV} (see Table 2) is given for *C*_{*a,*0} = 0.15 pM and *C*_{*a,*0} = 0.05 pM by the dashed black lines (see also Table 2). The panels on the right show zoom-ins of the sensor response at three different time periods (starting at *t* = 0 h, 12 h, and 42 h). In all cases the time-to-equilibrium is *τ* = 340 s = 5.7 minutes. Incubation with *C*_{*a,*0} = 0.15 pM gives depletion behavior at all times (since *f*ᵢₙᵢₜ < *f*_{eq*,IV*}, top black line); for *C*_{*a,*0} = 0.05 pM, depletion behavior is seen at *t <* 10 h and repletion at *t >* 10 h (when *f*ᵢₙᵢₜ > *f*_{eq*,IV*}, bottom black line).

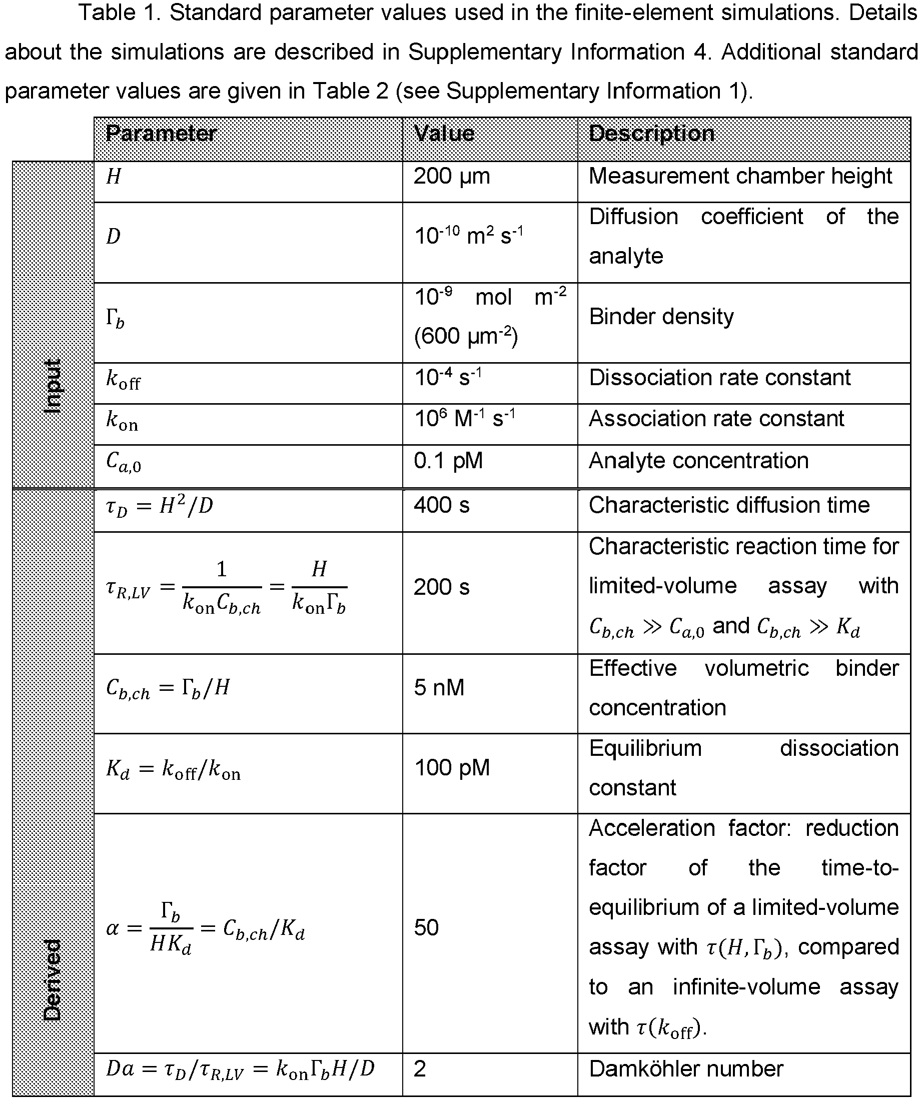

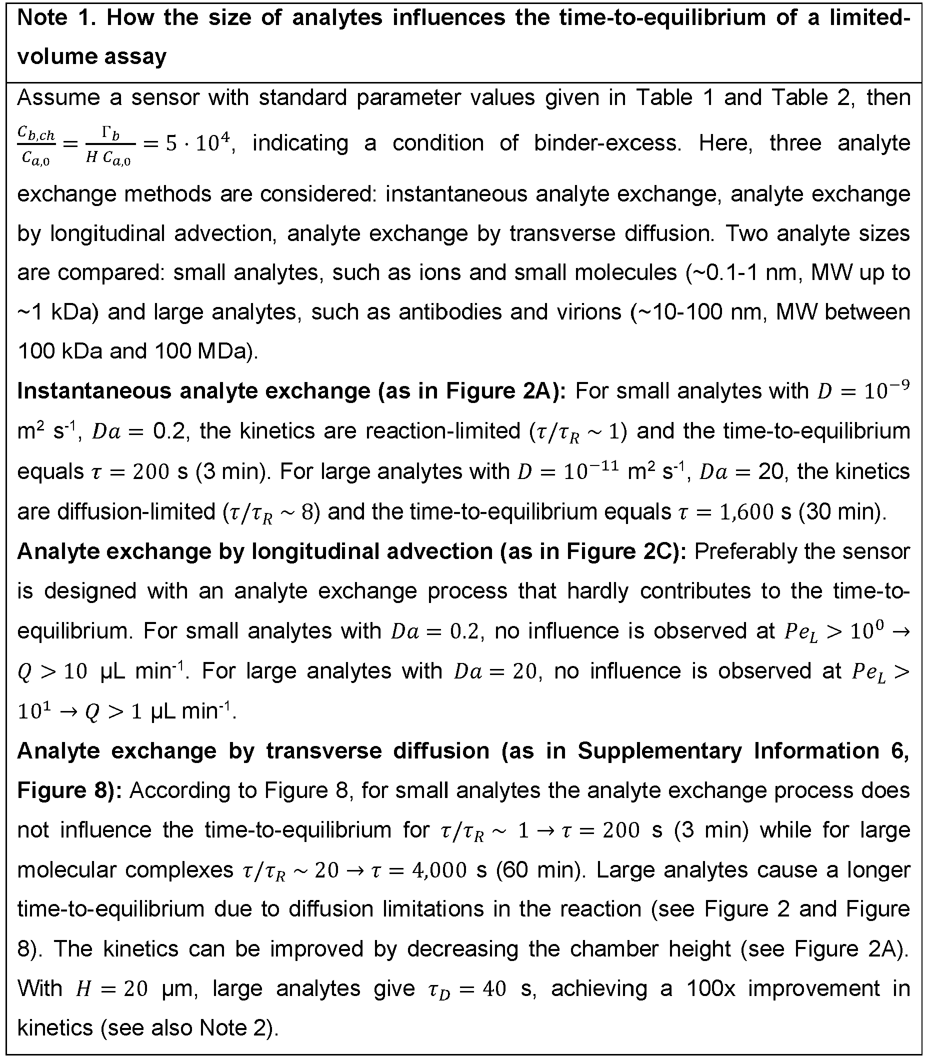

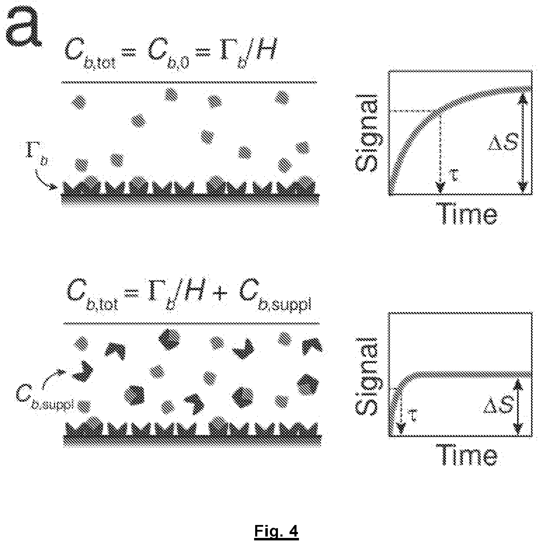

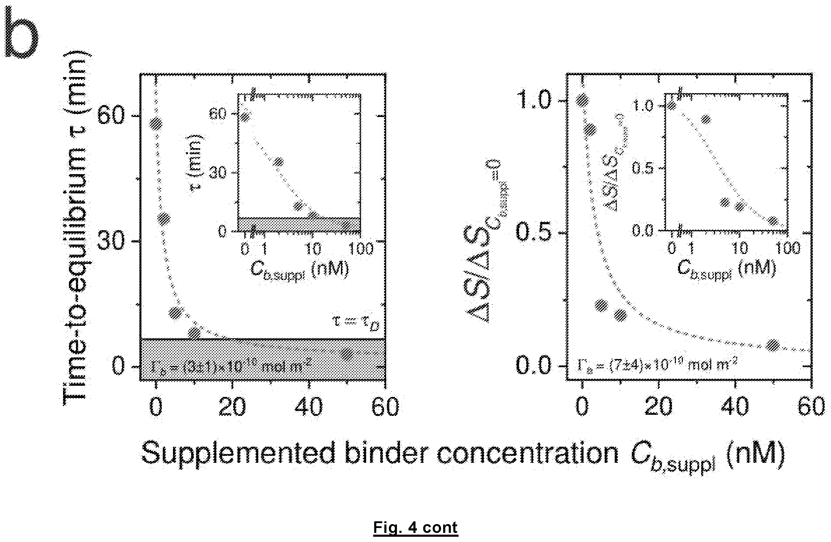

Figure 4 shows an experimental study on how the time-to-equilibrium in a limited-volume assay depends on the total binder concentration in the measurement chamber. Here, total binder concentration has two contributions, namely a contribution from surface-bound binders and a contribution from binders supplemented in solution. For detection use was made of Biosensing by Particle Mobility (BPM), which is an analyte monitoring principle with single-molecule resolution where the binding of analytes to specific binder molecules modulates the mobility of particles (see Supplementary Information 7). Figure 4A shows a schematic representation of a measurement chamber with binder molecules present in the two forms: immobilized and non-immobilized. Immobilized binder molecules are present with an effective volumetric concentration *C_{b,ch}*. Binder molecules supplemented free in solution have concentration *C*_{*b*,suppl}. In absence of supplemented binder molecules (top), the total binder concentration in the measurement chamber equals *C*_{*b,*tot} *= C_{b,ch} =* Γ*_{b}*/*H*. In presence of supplemented binder molecules (bottom), the total binder concentration equals *C*_{*b,*tot} *=* Γ*_{b}*/*H + C*_{*b*,suppl}. Since the time-to-equilibrium of the reaction scales according to *τ_{R,LV}* ∝ 1/*C*_{*b*,tot} (see Table 1), an increasing supplemented binder concentration *C*_{*b*,suppl} results in a smaller *τ*. Figure 4B shows the measured time-to-equilibrium *τ* (left) and the signal change ΔS (right) as a function of *C*_{*b*,suppl}, for an analyte concentration of 200 pM. The data show that the time-to-equilibrium decreases for increasing *C*_{*b*,suppl}. The measured signal change decreases with increasing supplemented binder concentration because only surface-captured analytes generate a measurable signal. The dashed lines in Figure 4B represent model fits (see the caption), demonstrating a good correspondence between model and experimental results. It was found that the measurements of Figure 4 prove the basic concept of the monitoring biosensor of the present invention, namely that a limited-volume design with time-controlled analyte exchange allows one to control the response time by tuning the concentration of binder molecules in the measurement chamber.

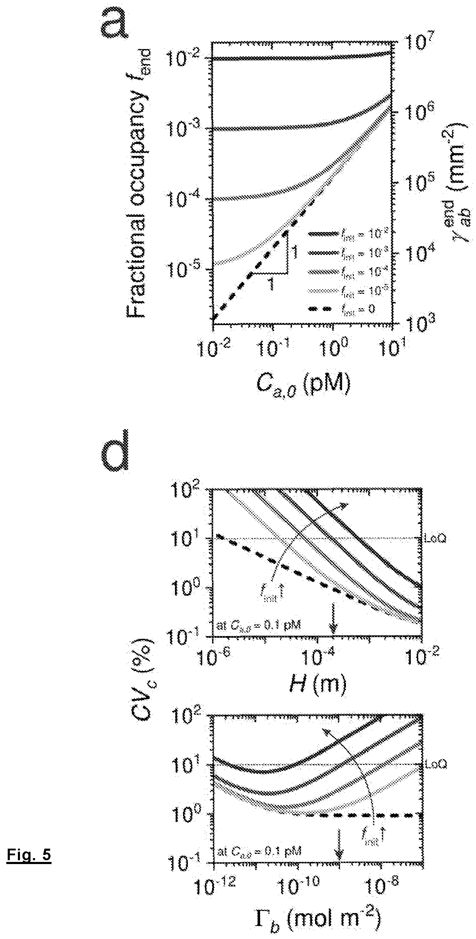

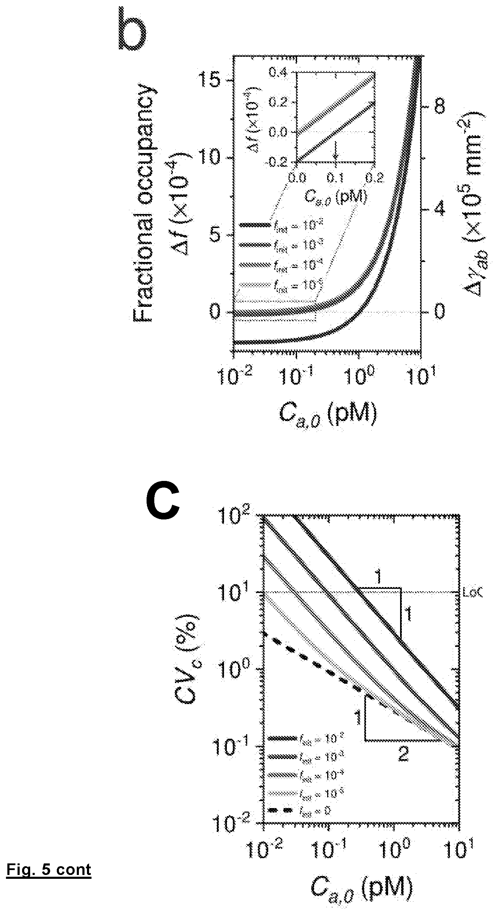

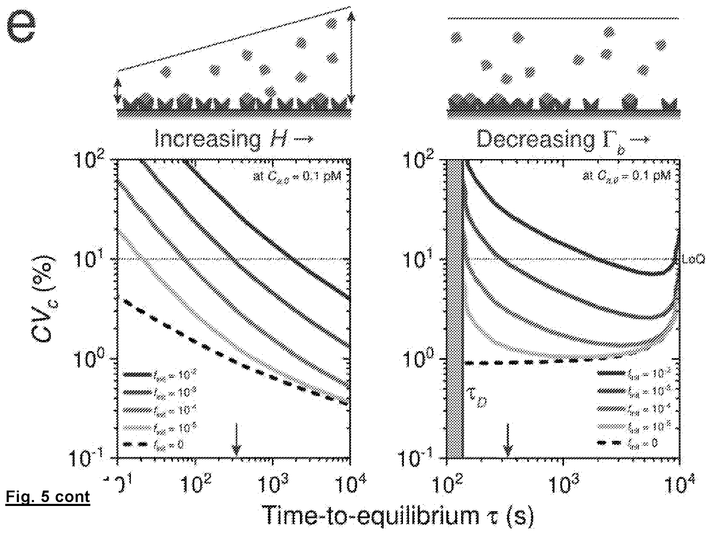

Figure 5 shows how the analytical performance of the limited-volume assay depends on the sensor design. The results are based on numerical simulations with parameters as listed in Table 1. The analyte exchange is assumed to be instantaneous and the secondary exchange phase includes mass transport by diffusion and reaction kinetics within the measurement chamber only. All panels show curves for different values of the initial fractional occupancy *f*ᵢₙᵢₜ of the binder molecules.

Figure 5A shows the fractional occupancy of binders by analytes at the end of the incubation (*f*_{end}) as a function of the input analyte concentration *C*_{*a*,0}. For *f*ᵢₙᵢₜ = 0 (dashed black line), *f*_{end} scales linearly with the analyte concentration, which makes the sensor suitable for analyte quantification. For larger values of *f*ᵢₙᵢₜ the curves start with a rather flat segment, from which one might erroneously conclude that under those conditions low analyte concentrations cannot be determined. Interestingly, the limited-volume assay has a linear dependence on concentration by focusing not on the *absolute* value of *f*_{end} but rather on the *change* of fractional occupancy Δ*f* (see Supplementary Information 2): $Δ f = {f}_{end} - {f}_{init} = \frac{1}{α + 1} \left(\frac{{C}_{a , o}}{{K}_{d}}-{f}_{init}\right) ≅ \frac{H}{{Γ}_{b}} \left({C}_{a , 0}-{K}_{d}{f}_{init}\right)$

This equation shows that *Δf* depends linearly on *C*_{*a*,0}, *independent* of the value of *f*ᵢₙᵢₜ. This fact is also illustrated by the simulation results in Figure 5B. The response scales linearly with concentration *C*_{*a,*0} and are down-shifted for increasing values of *f*ᵢₙᵢₜ, in agreement with Equation 1 (note that the steep increase of the curves relates to the logarithmic x-axis). Positive values of Δ*f* relate to depletion behavior and negative values to repletion. The curves cross the x-axis (Δ*f* = 0) when *f*ᵢₙᵢₜ corresponds to the equilibrium condition, *i.e.,* when there is no net association or dissociation during incubation because *f*ᵢₙᵢₜ is equal to the equilibrium fractional occupancy of the infinite-volume case: ${f}_{init} = {f}_{eq , IV} = \frac{{C}_{a , 0}}{{C}_{a , 0} + {K}_{d}} ≅ \frac{{C}_{a , 0}}{{K}_{d}}$ . For example, the curve for *f*ᵢₙᵢₜ = 10⁻³ crosses Δ*f* = 0 at *C*_{*a,*0} *= f*ᵢₙᵢₜ*K*_{d} = 0.1 pM, as is highlighted in the inset of Figure 5B.

Figure 5C shows the precision of the concentration output of the sensor, *i.e.,* the precision with which the analyte concentration in an unknown sample can be determined for a signal collection area of 1 mm². The precision is calculated based on Poisson noise, which gives the fundamental limit of the precision that is achievable with a molecular biosensor due to stochastic fluctuations in the number of analytes (see Supplementary Information 3 and 8). To calculate the precision, a sensor with initial fractional occupancy *f*ᵢₙᵢₜ is provided with a sample with analyte concentration *C*_{*a*,0}, resulting in a Δ*f* with variability *σ*_{Δ*f*}, which via the slope of the calibration curve, given in Figure 5B, leads to a variability *σ_{C}* in the concentration output of the sensor (see Supplementary Information 3). The precision is indicated as the concentration-based coefficient of variation *CV_{C} = σ_{C}*/*µ_{C}*, with *σ_{c}* the variability and *µ_{c}* the mean of the concentration output. Figure 5C shows how the concentration precision depends on the analyte concentration and the initial fractional occupancy *f*ᵢₙᵢₜ. For *f*ᵢₙᵢₜ = 0 (dashed line), the *CV_{C}* scales as $1 / \sqrt{{C}_{a , 0}}$, in agreement with number fluctuations in a Poisson process (see Supplementary Information 3). For higher *f*ᵢₙᵢₜ, a stronger dependency is observed (C*V_{C}* ∝ 1/*C*_{*a*,0}) caused by the smaller relative change of the fractional occupancy (see Supplementary Information 3). The graph indicates the 10% precision level that is used to define the limit of quantification (LoQ) of the sensor. The results show that analyte concentrations in the sub-picomolar range can be measured with a precision better than 10%, even for high initial fractional occupancies.

Figure 5D shows the precision of the concentration output of the sensor as a function of two design parameters, namely the measurement chamber height *H* (top panel) and the binder density Γ*_{b}* (bottom panel), at an analyte concentration *C*_{*a,*0} = 0.1 pM, for an initial fractional occupancy *f*ᵢₙᵢₜ between 0 and 0.01. The arrows indicate the height and density as listed in Table 1. For an increasing *H,* a decrease of *CV_{C}* is observed, caused by an increase in the number of analytes present in the measurement chamber. The *CV_{C}* is smallest for *f*ᵢₙᵢₜ = 0 and increases for increasing *f*ᵢₙᵢₜ since the absolute change of fractional occupancy decreases. The *CV_{C}* decreases for increasing Γ*_{b}* caused by an increase in the number of analytes captured from solution. The *CV_{C}* reaches a plateau for *f*ᵢₙᵢₜ = 0 due to a limited number of analytes in the measurement chamber. For larger *f*ᵢₙᵢₜ, the absolute change of fractional occupancy decreases and causes a less precise concentration determination; this effect is in particular visible at high Γ*_{b}* where the absolute number of analyte-binder complexes increases due to *f*ᵢₙᵢₜ.

The tradeoff between precision and time-to-equilibrium is illustrated in Figure 5E, for sensors with different heights of the measurement chamber (left) and different binder densities (right). The arrows indicate the time-to-equilibrium that results from the height and density as listed in Table 1. The left panel shows that an increase of *H* gives on the one hand a slower sensor response (due to a larger diffusion distance) but on the other hand a lower *CV_{C}* due to a larger number of analytes being exchanged by association and dissociation between the sensor surface and the measurement chamber. At low *H,* the *CV_{c}* strongly depends on *f*ᵢₙᵢₜ due to the low number of analytes in the solution. The right panel shows again that the *CV_{c}* decreases for a slower sensor response, now controlled by decreasing the binder density Γ*_{b}*. At high Γ*_{b}* the time-to-equilibrium is diffusion-limited (resulting in *τ* = 130 s). At low Γ*_{b}* the time-to-equilibrium is reaction-limited with *τ =* 1/*k*_{off} = 10⁴ (see Figure 2B). At high Γ*_{b}*, the *CV_{C}* increases for increasing *f*ᵢₙᵢₜ due to the larger amount of analytes on the sensor surface. At low Γ*_{b}*, the *CV_{C}* strongly increases due to the low number of captured molecules caused by analyte dissociation over the long incubation timescale.

| **Note 2. How the analyte size influences the sensitivity of the sensor.** |
|---|
| Assume a sensor as given in Note 1. As suggested in Note 1, with *H* = 20 µm, large analytes give *τ_{D}* = 40 s, achieving a 100x improvement of kinetics, but with a decrease in precision (see Figure 5D). Using *H* = 20 µm, for small analytes this would result in *CV_{c} =* 1% and for large analytes *CV_{c} =* 3%, but the precision decreases rapidly for increasing *f*ᵢₙᵢₜ (for example: *f*ᵢₙᵢₜ = 10⁻³ → *CV_{c} =* 10% and *CV_{c} =* 100% for small and large analytes respectively). The solution for this sharp decrease in precision is to decrease the binder density Γ*_{b}* (see Figure 5E, right) causing an increased precision with an increase in the time-to-equilibrium as a cost. |

Given the above, the present invention provides a sensing methodology suitable for monitoring low-concentration analytes with high sensitivity, with small time delays and short time intervals, over an endless time span. The sensing methodology is based on a limited-volume assay, using high-affinity binders, a reversible detection principle, and time-controlled analyte exchange. Based on simulations it was studied how the kinetics of the sensor depend on mass transport and on the surface reaction in the measurement chamber, and how time-controlled analyte exchange determines the system response. Experimental results show the ability to control the sensor response time by tuning the total binder concentration in the measurement chamber. Finally, simulations show that the sensing principle allows picomolar and sub-picomolar concentrations to be monitored with a high sensitivity over long time spans.

Approaches described in literature for measuring low-concentration analytes have focused primarily on assays in which every concentration determination involves consumption of reagents. When numbers of assays become high, due to frequent measurements over long time spans, then reagent consuming approaches are complex and costly. The sensing methodology of the present invention is based on a reversible assay principle, without consuming reagents with each newly recorded concentration datapoint, enabling measurements with high frequency over an endless time span. The described assay principle can be implemented on a variety of sensing platforms, *e.g*., based on optical, electrical, or acoustical transduction methods, where especially sensing platforms with single-molecule resolution seem suitable since these will allow digital measurements with the highest sensitivity and therefore shortest response times. The described assay principle can be combined with a variety of sampling methods, including remote advection-based sampling and proximal diffusion-based sampling methods. Due to its generalizability and unique and tunable sensing performance, it was found that the limited-volume assay with time-controlled analyte exchange enables studies on time-dependencies of low-concentration analytes and novel applications in the fields of dynamic biological systems, patient monitoring, and biotechnological process control.

### Experimental section

The experimental section comprises the method of the simulations performed, example calculations and supplementary information referred to throughout the application.

### Finite-element analysis.

Finite-element simulations were performed by solving diffusion, advection and reaction equations simultaneously using COMSOL (COMSOL Multiphysics 5.5) and MATLAB (MATLAB R2019a, COMSOL Multiphysics LiveLink for MATLAB) (see Supplementary Information 4). From the simulations, the time-to-equilibrium *τ* was determined by calculating the time at which the analyte-binder complexes *γ_{ab}* is at 63% of the difference between the starting level and the equilibrium level of *γ_{ab}*. The time-controlled analyte exchange (see Figure 3) was simulated by instantaneously increasing/decreasing the analyte concentration in the measurement chamber *Cₐ* to *C*_{*a*,0}, with which a new measurement cycle starts. The density of analyte-binder complexes ${γ}_{ab}^{start}$ at the start of a cycle was set to be equal to the density of analyte-binder complexes ${γ}_{ab}^{end}$ at the end of the preceding cycle. The infinite-volume assay was simulated by forcing the analyte concentration in the measurement chamber *Cₐ* to be equal to *C*_{*a*,0}. Sensor signals are reported at distance *L*/2 in the measurement chamber (see Figure 1D). Precisions are reported at a distance *L*/2 in the measurement chamber, where the signal is collected over a signal collection area of 1 mm² (Figure 5C-Figure 5E).

### Fluid cell assembly

Glass slides (25 x 75 mm, #5, Menzel-Gläser) were cleaned by 40 minutes sonication in isopropanol (VWR, absolute) and twice by 10 minutes sonication in MilliQ (ThermoFischer Scientific, Pacific AFT 20). Subsequently, the glass slides were dried under nitrogen flow. A polymer mixture of PLL(20)-g[3.5]-PEG(2) (SuSoS) and PLL(15)-g[3.5]-PEG(2)-N₃ (Nanosoft Polymers) was prepared at a final concentration of 0.45 mg mL⁻¹ and 0.05 mg mL⁻¹ in MilliQ respectively. The glass slides were treated by oxygen plasma (Plasmatreat GmbH) for 1 minute. A custom-made fluid cell sticker (Grace Biolabs), with an approximate volume of 20 µL, was attached to the glass slide and immediately filled with the polymer mixture. After 2 hour incubation, the polymer mixture was removed and the fluid cell was immediately filled with 0.5 nM dsDNA tether solution (221 bp, with DBCO at one end and biotin at the other end) in 0.5 M NaCl in PBS. After overnight incubation, the solution in the fluid cell was exchanged by 2 µM DBCO-functionalized dsDNA solution in 0.5 M NaCl in PBS and incubated for several days until use.

### Particle functionalization

2 µL streptavidin-functionalized particles (10 mg/mL, Dynabeads MyOne Streptavidin C1, Thermo Fisher Scientific) was incubated with 1 µL biotinylated ssDNA binder molecules (10 µM, IDT, HPLC purification) and 4 µL PBS for 70 minutes. The particles were magnetically washed in 5 vol.-% Tween-20 (Sigma-Aldrich) in PBS and resuspended in 0.5 M NaCl in PBS to a final concentration of 0.1 mg/mL and sonicated using an ultrasonic probe (Hielscher).

### BPM assay

25 µL particle solution was added to the fluid cell and incubated for 10 minutes. After incubation, the fluid cell was reversed causing unbound particles to sediment. After washing with 40 µL 0.5 M NaCl in PBS, 40 µL mPEG-biotin (500 µM, PG1-BN-1k, Nanocs) in 0.5 M NaCl in PBS was added to the fluid cell. After 15 minutes incubation, the fluid cell was washed twice with 40 µL PBS. A mixture of ssDNA analytes (IDT, standard desalting) and free binder molecules in PBS was added to the flow cell at the required concentration, immediately after preparation. The sample was observed under a white light source using a microscope (Leica DMI5000M) with a dark field illumination setup at a total magnification of 10× (Leica objective, N plan EPI 10x/0.25 BD). A field of view of approximately 1100×700 µm² was imaged using a CMOS camera (FLIR, Grasshopper3, GS3-U3-23S6M-C) with an integration time of 5 ms and a sampling frequency of 30 Hz. The particles were tracked by applying a phasor-based localization method. The particle activity was determined from the x- and y-trajectories of all particles, by applying a maximum-likelihood multiple-windows change point detection algorithm. The particle activity at equilibrium and the time-to-equilibrium were extracted by fitting the measured particle activity over time using the equation given in Note 3.

### Supplementary Information 1: standard parameter values

Standard parameter values used throughout the application and the Supplementary Information are listed in Table 2.

### Supplementary Information 2: analytical expression of the dose-response curve

In a limited-volume sensor with time-controlled analyte exchange, a limited number of analytes interact with binder molecules present in a measurement volume. The input analyte concentration *C*_{*a,*0} can be derived from the time-evolution of the density of surface-bound analyte-binder complexes *γ_{ab}*. It is assumed that all binder molecules are immobilized on a surface, with effective volumetric concentration *C_{b,ch} =* Γ*_{b}*/*H* where Γ_{b} is the density of surface binders and *H* the height of the measurement chamber. It is assumed that binder molecules are in excess compared to analytes (*C_{b,ch}* > *C*_{*a*,0}) and are in excess compared to the equilibrium dissociation constant (*C_{b,ch} > K_{d}*). Furthermore, it is assumed that during the secondary exchange phase no analytes are exchanged between the system of interest and the measurement chamber. Assuming first-order Langmuir kinetics, the change in effective volumetric analyte-binder complex concentration per unit time can be determined by: $\frac{{dC}_{ab} \left(t\right)}{dt} = {k}_{on} \left({C}_{a , 0}-{C}_{ab}\left(t\right)+{f}_{init}{C}_{b , ch}\right) {C}_{b , ch} - {k}_{off} {C}_{ab} \left(t\right)$ with $\frac{{dC}_{ab} \left(t\right)}{dt}$ being the time-derivative of the (spatial-dependent) effective volumetric analyte-binder complex concentration *C_{ab}, kₒₙ* the association rate constant, *C*_{*a,*0} the input analyte concentration, *f*ᵢₙᵢₜ the initial fractional occupancy of the binder by an analyte, *C_{b,ch}* the total effective binder concentration, and *k_{off}* the dissociation rate constant. Using *C_{ab}*(*t*) *= γ_{ab}*(*t*)/*H*, where *γ_{ab}* is the density of analyte-binder complexes, Equation S1 can be rewritten as a surface reaction rate: $\frac{{dγ}_{ab} \left(t\right)}{dt} = {k}_{on} \left({C}_{a , 0}+{f}_{init}\frac{{Γ}_{b}}{H}\right) {Γ}_{b} - {k}_{on} \left(\frac{{Γ}_{b}}{H}+{K}_{d}\right) {γ}_{ab} \left(t\right)$ with $\frac{{dγ}_{ab} \left(t\right)}{dt}$ being the time-derivative of the density *γ_{ab}* of analyte-binder complexes, Γ*_{b}* the binder density, and *K_{d}* the equilibrium dissociation constant. To calculate the time-dependent response of the sensor, the differential equation was solved given in Equation S2 in Note 3 and get the general solution for the time-evolution of the density *γ_{ab}* of analyte-binder complexes after instantaneous analyte exchange and where no mass transport effects are considered.

| **Note 3. Derivation of the analytical expression for the time-evolution of the density *γ_{ab}* of analyte-binder complexes after instantaneous analyte exchange** |
|---|
| $\frac{{dγ}_{ab} \left(t\right)}{dt} = {k}_{on} \left({C}_{a , 0}+{f}_{init}\frac{{Γ}_{b}}{H}\right) {Γ}_{b} - {k}_{on} \left(\frac{{Γ}_{b}}{H}+{K}_{d}\right) {γ}_{ab} \left(t\right)$ |
| $\begin{matrix}\frac{{dγ}_{ab} \left(t\right)}{dt} + {C}_{1} {γ}_{ab} \left(t\right) = {C}_{2} \to {γ}_{ab} \left(t\right) = \frac{{C}_{2}}{{C}_{1}} + {C}_{3} {e}^{- At} = \frac{{Γ}_{b}}{{Γ}_{b} / H + {K}_{d}} \left({C}_{a , 0}+{f}_{init}\frac{{Γ}_{b}}{H}\right) + \\ {C}_{3} {e}^{- {k}_{on} \left({Γ}_{b}/H+{K}_{d}\right) t}\end{matrix}$ |
| ${γ}_{ab} \left(t=0\right) = {f}_{init} {Γ}_{b} = \frac{{Γ}_{b}}{{Γ}_{b} / H + {K}_{d}} \left({C}_{a , 0}+{f}_{init}\frac{{Γ}_{b}}{H}\right) + {C}_{3} \to {C}_{3} = {f}_{init} {Γ}_{b} - \frac{{Γ}_{b}}{{Γ}_{b} / H + {K}_{d}} \left(\begin{matrix}{C}_{a , 0} + \\ {f}_{init} \frac{{Γ}_{b}}{H}\end{matrix}\right)$ |
| ${γ}_{ab} \left(t\right) = \frac{{Γ}_{b}}{{Γ}_{b} / H + {K}_{d}} \left({C}_{a , 0}+{f}_{init}\frac{{Γ}_{b}}{H}\right) + \left({f}_{init}{Γ}_{b}-\frac{{Γ}_{b}}{{Γ}_{b} / H + {K}_{d}}\left({C}_{a , 0}+{f}_{init}\frac{{Γ}_{b}}{H}\right)\right) {e}^{- {k}_{on} \left({Γ}_{b}/H+{K}_{d}\right) t}$ |
| ${γ}_{ab} \left(t\right) = \frac{α}{α + 1} \left({HC}_{a , 0}+{f}_{init}{Γ}_{b}\right) + {βe}^{- t / {τ}_{R}}$ |

Here, *α =* Γ*_{b}*/(*HK_{d}*) is the acceleration factor (see Table 2), $β = {f}_{init} {Γ}_{b} - \frac{α}{α + 1} \left({HC}_{a , 0}+{f}_{init}{Γ}_{b}\right)$ , and ${τ}_{R} = {\left({k}_{on}\left(\frac{{Γ}_{b}}{H}+{K}_{d}\right)\right)}^{- 1}$ is the characteristic time-to-equilibrium of the reaction. When the *γ_{ab}*reaches equilibrium (*i.e*., *t* → ∞), then ${γ}_{ab} = {γ}_{ab}^{and} = \frac{α}{α + 1} \left({HC}_{a , 0}+{f}_{init}{Γ}_{b}\right)$ . For a limited-volume sensor with *C_{b,ch} > C*_{*a,*0} and *C_{b,ch} > K_{d}* in the equilibrium condition, nearly all analytes are bound ( ${f}_{bound} = \frac{α}{α + 1} ≅ 1$) and only a small fraction of analytes is unbound ( ${f}_{unbound} = \frac{1}{α + 1}$). Therefore *τ_{R}* can be simplified to *τ_{R}* = *H*/(*k*ₒₙΓ*_{b}*).

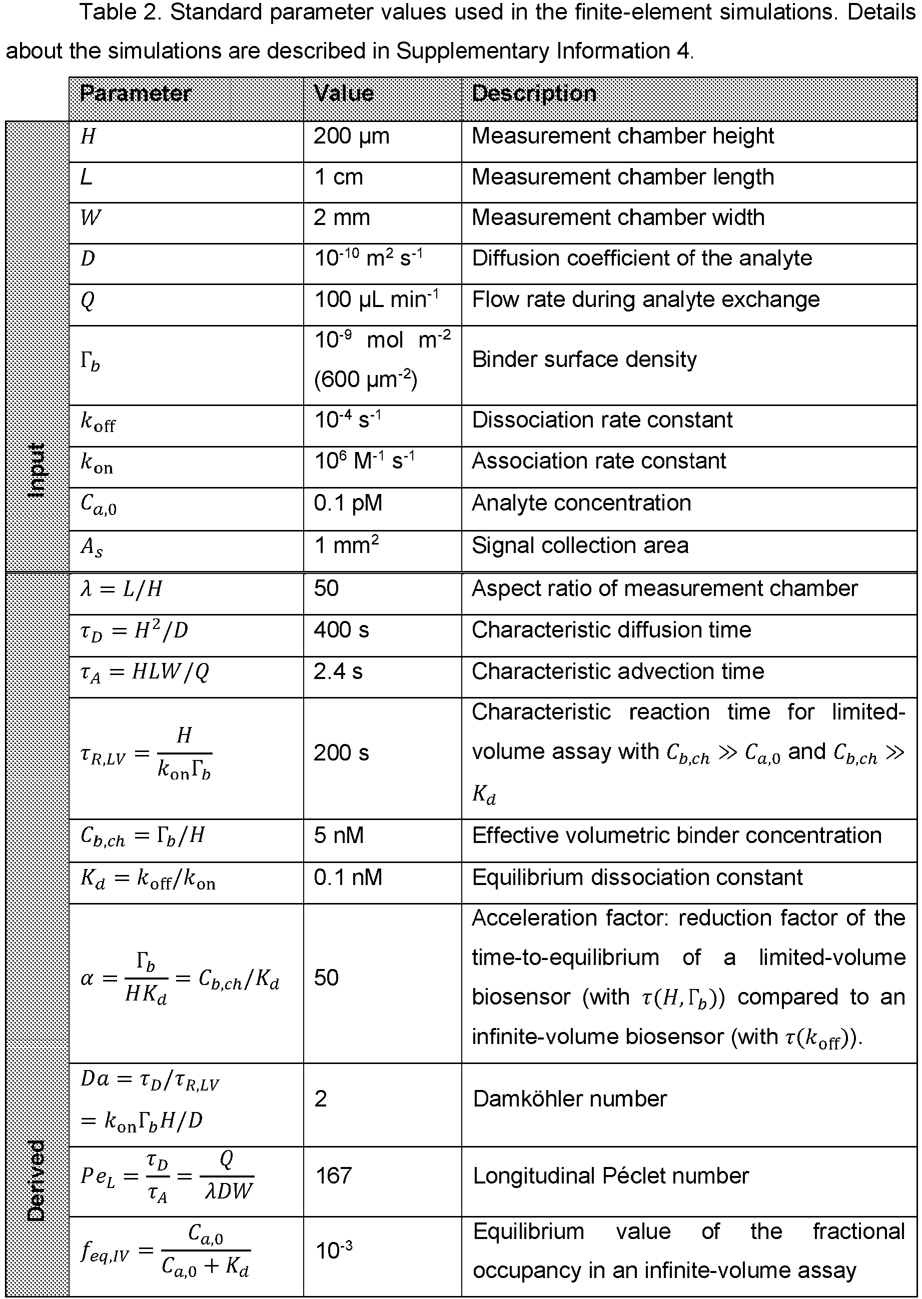

In the equation of the time-evolution of the density of analyte-binder complexes (see Note 3), the depletion and repletion regimes can be recognized. When *f*ᵢₙᵢₜ < *C*_{*a,*0}/ *K_{d}*, then *β* < 0, so the sensor shows depletion behavior. Conversely, if *f*ᵢₙᵢₜ *> C*_{*a,*0}/*K_{d}*, then *β* > 0 and the sensor shows repletion behavior. Rewriting the time-evolution of the density of analyte-binder complexes using the fractional occupancy *f = γ_{ab}*/Γ*_{b}* and *t* → ∞, yields the dose-response relationship as visualized in Figure 5A: ${f}_{end} = \frac{α}{α + 1} \left(\frac{H}{{Γ}_{b}}{C}_{a , 0}+{f}_{init}\right) ≅ \frac{H}{{Γ}_{b}} {C}_{a , 0} + {f}_{init}$

Therefore *f*_{end} depends linearly on *C*_{*a*,0}, independent of the value of *f*ᵢₙᵢₜ. The *change* of fractional occupancy Δ*f* yields the dose-response relation as visualized in Figure 5B: $Δ f = {f}_{end} - {f}_{init} = \frac{α}{α + 1} \left(\frac{H}{{Γ}_{b}}{C}_{a , 0}+{f}_{init}\right) - {f}_{init} = \frac{1}{α + 1} \left(\frac{{C}_{a , 0}}{{K}_{d}}-{f}_{init}\right) ≅ \frac{H}{{Γ}_{b}} \left({C}_{a , 0}-{K}_{d}{f}_{init}\right)$

Here, *Δf* depends linearly on *C*_{*a*,0}, independent of the value of *f*ᵢₙᵢₜ.

### Supplementary Information 3: sensitivity

The precision of the concentration output of the sensor, is the precision with which the analyte concentration in an unknown sample can be determined using the limited-volume assay. Under the assumption that the measured signal change Δ*S* = *S*ᵢₙᵢₜ - *S*_{end} scales linearly with the change in fractional occupancy Δ*f*, *i.e*., Δ*S* ∝ Δ*f*, the precision of *C*_{*a,*0} is calculated using the precision with which *S*ᵢₙᵢₜ and *S*_{end} can be determined.

It was assumed that measurement variabilities are dominated by Poisson noise in the number of bound analytes; other factors contributing to variability are not taken into account. An analytical expression is derived for the precision of the analyte concentration *C*_{*a,*0} in Note 4 of which the results are given in Figure 5C-Figure 5E.

| **Note 4. Derivation of the analytical expression for the precision of the analyte concentration *C*_{*a,*0} based on Poisson noise only** |
|---|
| The error of the signal *σ_{S}* without background can be estimated by: |
| ${σ}_{{N}_{ab}^{end}} = \sqrt{{N}_{ab}^{end}} and {σ}_{{N}_{ab}^{init}} = \sqrt{{N}_{ab}^{init}}$ |
| where ${N}_{ab}^{end}$ and ${N}_{ab}^{init}$ are the total number of analytes bound to binder molecules that contribute to the signal of the sensor, at the end and start of a measurement respectively. |
| The measurement signal change equals Δ*S* = *S*_{end} - *S*ᵢₙᵢₜ. Thus the squared signal change error is equal to the sum of the squared errors of the two terms: |
| ${{σ}_{Δ S}}^{2} = {σ}_{{S}_{end}}^{2} + {σ}_{{S}_{init}}^{2} = {\left(\frac{{S}_{end}}{\sqrt{{N}_{ab}^{end}}}\right)}^{2} + {\left(\frac{{S}_{init}}{\sqrt{{N}_{ab}^{init}}}\right)}^{2}$ |
| The concentration of the analyte is the output of the sensor. The output precision can be determined using the signal change error and the slope of the calibration curve, *i.e.,* the slope of the dose-response curve: ${σ}_{C} = \frac{{δC}_{a , 0}}{δ Δ S} {σ}_{Δ S}$ with $\frac{δ Δ S}{{δC}_{a , 0}}$ the concentration-derivative of the signal change. This gives the following expression for the error of the concentration determined using a sensor with Poisson-limited precision: |
| ${σ}_{C} = \frac{{δC}_{a , 0}}{δ Δ S} \sqrt{{\left(\frac{{S}_{end}}{\sqrt{{N}_{ab}^{end}}}\right)}^{2} + {\left(\frac{{S}_{init}}{\sqrt{{N}_{ab}^{init}}}\right)}^{2}}$ |

The derivation in Note 4 gives the following analytical expression for the precision of the sensor output, *i.e.,* the error of the concentration *σ_{C}*: ${σ}_{C} = \frac{{δC}_{a , 0}}{δ Δ S} \sqrt{{\left(\frac{{S}_{end}}{\sqrt{{N}_{ab}^{end}}}\right)}^{2} + {\left(\frac{{S}_{init}}{\sqrt{{N}_{ab}^{init}}}\right)}^{2}}$

Equation S5 shows that *σ_{C}* decreases (*i.e.,* the precision increases) for an increasing number of analytes ${N}_{ab}^{end}$ for a given signal collection area (see Table 2), for instance by increasing the height of the measurement chamber or the binder density (see Note 3). Since *S*_{end} and thus ${N}_{ab}^{end}$ scale linearly with the analyte concentration (see Equation S3), the following can be derived: ${σ}_{C} ∝ \sqrt{{C}_{a , 0}} \to {CV}_{C} = \frac{{σ}_{C}}{{C}_{a , 0}} ∝ \frac{\sqrt{{C}_{a , 0}}}{{C}_{a , 0}} = \frac{1}{\sqrt{{C}_{a , 0}}}$ which results in a 1:2 slope (*CV_{C}*:*C*_{*a.*0}) in Figure 5A for low *f*ᵢₙᵢₜ. However, if *f*ᵢₙᵢₜ is close to or higher than *HC*_{*a,*0}/Γ*_{b}*, then the contribution of *f*ᵢₙᵢₜ to the fractional occupancy *f*_{end} at the end of a measurement cycle is relatively large, which results in a rather flat segment in the *f*_{end}-*C*_{*a*,0} curve as visualized in Figure 5A. Since *f*_{end} ~ *f*ᵢₙᵢₜ, *σ_{C}* is largely determined by *f*ᵢₙᵢₜ, then Equation S6 converts to ${CV}_{C} = \frac{{σ}_{C}}{{C}_{a , 0}} ∝ \frac{1}{{C}_{a , 0}}$, resulting in a 1:1 slope (*CV_{C}*:*C*_{*a.*0}) in Figure 5C for high *f*ᵢₙᵢₜ.

### Supplementary Information 4: nondimensionalization

The simulation study of the time-dependent behavior of the biochemical assay was performed using dimensionless parameters for all mass transport processes and reaction rates. The nondimensionalized parameters for mass transport by diffusion and advection are given in Table 3.

**Table 3. Dimensionless parameters used in the finite-element analysis for modeling mass transport by diffusion and advection.**

| **Dimensionless parameter** | **Symbol** | **Expression** |
|---|---|---|
| Analyte concentration | *c̃* | *c̃* = *Cₐ*/*C*_{*a*,0} |
| Longitudinal distance | *x̃* | *x̃ = x*/*L* |
| Transversal distance | *ỹ* | *ỹ = y*/*H* |
| Time | *t̃* | *t = H²*/*D* → *t̃ = t D*/*H*² |

For all finite-element analyses, the time was nondimensionalized using the diffusion time *τ_{D}* (*e.g*., Figure 2B) and thereafter recalculated to normalize with respect to other time scales (*e.g*., *τ_{R}* in Figure 2A and Figure 2C). When advective flow is included, the used analytical expression of the advective flow is given by: $\vec{v} \left(y\right) = \frac{6 Q}{{WH}^{3}} y \left(H-y\right) \vec{{e}_{x}}$
with *̅v̅*̅(*y*) the flow speed as a function of the height inside the measurement chamber *y*, *Q* the flow rate, *W* the width of the measurement chamber, and *H* the height of the measurement chamber. The general equation used in the simulation to describe mass transport by advection and diffusion is given by: $\frac{{δC}_{a}}{δt} = D {∇}^{2} {C}_{a} - \vec{v} \left(y\right) ⋅ ∇ {C}_{a}$
with $\frac{{δC}_{a}}{δt}$ being the time-derivative of the (spatial-dependent) analyte concentration *Cₐ* and *D* the diffusion coefficient. The dimensionless form of Equation S8 using the defined parameters in Table 3 is derived in Note 5.

| **Note 5. Derivation of the dimensionless advection-diffusion equation** |
|---|
| $\frac{δ \left({c}^{˜}{C}_{a . 0}\right)}{δ \left(\frac{{t}^{˜} {H}^{2}}{D}\right)} = D \frac{{δ}^{2} \left({c}^{˜}{C}_{a . 0}\right)}{δ {\left({x}^{˜}L\right)}^{2}} + D \frac{{δ}^{2} \left({c}^{˜}{C}_{a . 0}\right)}{δ {\left({y}^{˜}H\right)}^{2}} - \frac{6 Q}{{WH}^{3}} {y}^{˜} H \left(H-{y}^{˜}H\right) \frac{δ \left({c}^{˜}{C}_{a . 0}\right)}{δ \left({x}^{˜}L\right)}$ |
| $\frac{δ {c}^{˜}}{δ {t}^{˜}} = \frac{{H}^{2}}{D} \frac{1}{{L}^{2}} D \frac{{δ}^{2} {c}^{˜}}{δ {{x}^{˜}}^{2}} + \frac{{H}^{2}}{D} \frac{1}{{H}^{2}} D \frac{{δ}^{2} {c}^{˜}}{δ {{y}^{˜}}^{2}} - \frac{{H}^{2}}{D} \frac{6 Q}{{WH}^{3}} \frac{1}{L} {y}^{˜} {H}^{2} \left(1-{y}^{˜}\right) \frac{δ {c}^{˜}}{δ {x}^{˜}}$ |
| $\frac{δ {c}^{˜}}{δ {t}^{˜}} = \frac{{H}^{2}}{{L}^{2}} \frac{{δ}^{2} {c}^{˜}}{δ {{x}^{˜}}^{2}} + \frac{{δ}^{2} {c}^{˜}}{δ {{y}^{˜}}^{2}} - \frac{6 QH}{LDW} {y}^{˜} \left(1-{y}^{˜}\right) \frac{δ {c}^{˜}}{δ {x}^{˜}}$ |

Using the derivation given in Note 5, measurement chamber aspect ratio *λ = L*/*H*, and longitudinal Péclet number ${Pe}_{L} = \frac{Q}{λDW}$ (see Table 2), the simplified dimensionless advection-diffusion equation is given by: $\frac{δ {c}^{˜}}{δ {t}^{˜}} = \frac{1}{{λ}^{2}} \frac{{δ}^{2} {c}^{˜}}{δ {{x}^{˜}}^{2}} + \frac{{δ}^{2} {c}^{˜}}{δ {{y}^{˜}}^{2}} - 6 {Pe}_{L} {y}^{˜} \left(1-{y}^{˜}\right) \frac{δ {c}^{˜}}{δ {x}^{˜}}$

The nondimensionalized parameters for the reaction rate are given in Table 4.

**Table 4. Dimensionless parameters used in the finite-element analysis for modeling the reaction at the sensor surface.**

| **Dimensionless parameter** | **Symbol** | **Expression** |
|---|---|---|
| Analyte concentration at the sensor surface | *c̃** | ${c}^{˜} * = {C}_{a}^{∗} / {C}_{a , 0}$ |
| Density of analyte-binder complexes | *γ̃* | *γ̃* = *γ_{ab}*/(*C*_{*a,*0}*H)* |
| Time | *t̃* | *t̃ = T D*/*H*² |

The general equation used in the simulation to model the reaction at the sensor surface is given by: $\frac{{δγ}_{ab} \left(t\right)}{δt} = {k}_{on} {C}_{a}^{∗} \left({Γ}_{b}-{γ}_{ab}\left(t\right)\right) - {k}_{off} {γ}_{ab} \left(t\right)$ with $\frac{{δγ}_{ab}}{δt}$ the time-derivative of the (spatial-dependent) density *γ_{ab}* of analyte-binder complexes and ${C}_{a}^{∗}$ the analyte concentration at the sensor surface, which is known by solving Equation S9. The dimensionless form of Equation S11 is derived in Note 6.

| **Note 6. Derivation of the dimensionless reactive rate equation** |
|---|
| $\frac{δ \left({γ}^{˜}{C}_{a , 0}H\right)}{δ \left(\frac{{{t}^{˜} H}^{2}}{D}\right)} = {k}_{on} {c}^{˜} * {C}_{a , 0} \left({Γ}_{b}-{γ}^{˜}{C}_{a , 0}H\right) - {k}_{off} {γ}^{˜} {C}_{a , 0} H$ |
| $\frac{δ {γ}^{˜}}{δ {t}^{˜}} = \frac{{H}^{2}}{D} \frac{{k}_{on} {c}^{˜} *}{H} \left({Γ}_{b}-{γ}^{˜}{C}_{a , 0}H\right) - \frac{{H}^{2}}{D} {k}_{off} {γ}^{˜}$ |
| $\frac{δ {γ}^{˜}}{δ {t}^{˜}} = \frac{{k}_{on} {Γ}_{b} H}{D} {c}^{˜} * - \frac{{k}_{on} H}{D} {c}^{˜} * {γ}^{˜} {C}_{a , 0} H - \frac{{H}^{2}}{D} {k}_{off} {γ}^{˜}$ |
| $\frac{δ {γ}^{˜}}{δ {t}^{˜}} = \frac{{C}_{a , 0} H}{{Γ}_{b}} \frac{{k}_{on} {Γ}_{b} H}{D} \left[{c}^{˜}*\left(\frac{{Γ}_{b}}{{C}_{a , 0} H}-{γ}^{˜}\right)-\frac{{K}_{d}}{{C}_{a , 0}}{γ}^{˜}\right]$ |

Using the derivation given in Note 6 and Damköhler number $Da = \frac{{k}_{on} {Γ}_{b} H}{D}$ (see Table 2), the simplified dimensionless reactive rate equation is given by: $\frac{δ {γ}^{˜}}{δ {t}^{˜}} = \frac{{C}_{a , 0} H}{{Γ}_{b}} Da \left[{c}^{˜}*\left(\frac{{Γ}_{b}}{{C}_{a , 0} H}-{γ}^{˜}\right)-\frac{{K}_{d}}{{C}_{a , 0}}{γ}^{˜}\right]$

### Supplementary Information 5: time-controlled analyte exchange

Time-controlled analyte exchange in a limited-volume assay refers to the switching between the primary exchange phase and the secondary exchange phase (see Figure 1D). In the primary exchange phase, analytes are exchanged effectively between the system of interest and the measurement chamber, *e.g*., by diffusion and/or advection. In the secondary exchange phase, analytes are exchanged ineffectively between the system of interest and the measurement chamber, causing a limited-volume incubation in the measurement chamber. The analyte exchange can be controlled in time by changing the mass transport between the system of interest and the measurement chamber, *e.g*., by stopping flow and/or diffusion. It is assumed that there is no analyte exchange during the secondary exchange phase (*i.e*., *τ_{sec.exch}* → ∞) causing time scale *τ_{sec.exch}* and duration *t_{sec.exch}* to be irrelevant; therefore the characteristic time of primary exchange is indicated *by τ_{exch}* and the duration of the primary exchange is indicated by *t_{exch},* both without the primary subscript.

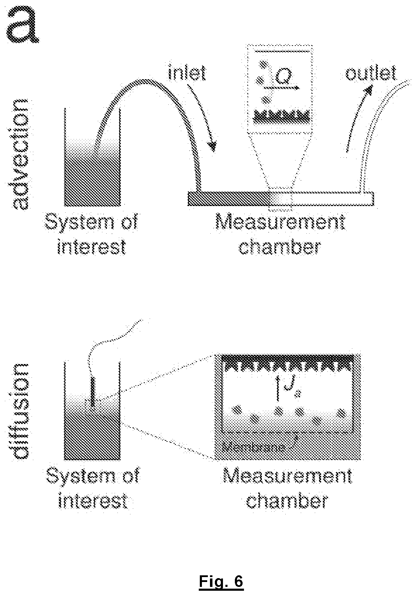

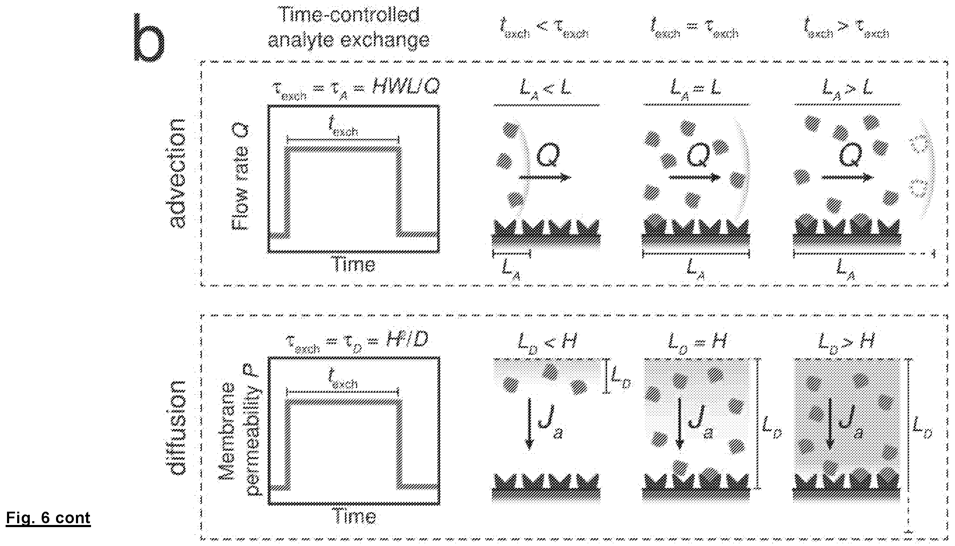

Figure 6 shows how time-controlled analyte exchange influences the performance of the sensor, for two analyte exchange principles, namely remote advection-based sampling and proximal diffusion-based sampling. In Supplementary Information 6, the influence of time-controlled analyte exchange on the performance of the sensor is quantified by simulating the time-to-equilibrium *τ* and the coefficient of variation of the concentration *CV_{C}* as a function of the duration of the primary exchange phase *t_{exch}*.

Figure 6A schematically visualizes time-controlled analyte exchange by advection (top) and by diffusion (bottom), where the primary exchange has a characteristic time *τ_{exch}* and a duration *t*_{exch} (see Figure 6B). *t*_{exch} is controlled by controlling the flow rate *Q* or the molar flux *Jₐ* (by controlling the membrane permeability *P*) in time.³ For advection-based sampling, *τ*_{exch} equals the characteristic advection time *τ_{A}* (see Table 2), while for diffusion-based sampling, *τ*_{exch} equals the characteristic diffusion time *τ_{D}* (see Table 2). In *t*_{exch}, analytes are transported over a characteristic length *L_{A} = t*_{exch}*Q*/(*HW*) by advection and ${L}_{D} = \sqrt{{t}_{exch} D}$ by diffusion.

Three regimes can be identified with regard to the analyte exchange. First, *t*_{exch} < *τ*_{exch} implies that *L_{A}* or *L_{D}* is shorter than the length *L* of the measurement chamber or the height *H* of the measurement chamber for advection-based sampling and diffusion-based sampling respectively. Second, If *t*_{exch} equals *τ*_{exch}, the transport distance equals *L* or *H* for advection-based sampling and diffusion-based sampling respectively; this condition is used in Figure 2C for analyte exchange by advection, since here the exchanged volume equals the volume of the measurement chamber. Third, *t*_{exch} > *τ*_{exch} implies that *L_{A}* or *L_{D}* is longer than *L* or *H* for advection-based sampling and diffusion-based sampling respectively.

In the simulations the following assumptions were made. First, analyte exchange between the system of interest and the measurement chamber only occurs during the primary exchange phase. For advection-based analyte exchange, this implies that the flow rate *Q* is high in phase 1 (the primary exchange phase) and zero in phase 2 (the secondary exchange phase). For diffusion-based analyte exchange, the membrane permeability *P* is high in phase 1, and zero in phase 2. The second assumption is that diffusive mass transport within the measurement chamber itself occurs at all times.

Analyte exchange can be controlled by controlling the characteristic analyte exchange time *τ*_{exch} (by design parameters *Q* and *P*) and by controlling the analyte exchange duration *t*_{exch}. In Supplementary Information 6 the performance as a function of *Q* and *t*_{exch} for advection-based exchange is studied. For diffusion-based exchange, the performance as a function of *t*_{exch} is studied, assuming *P* = 0 or *P* → ∞.

The ratio between mass transport facilitated by diffusion *versus* mass transport facilitated by advection can be compared using the longitudinal Péclet number *Pe_{L}:* ${Pe}_{L} = \frac{{k}_{A}}{{k}_{D}} = \frac{{τ}_{D}}{{τ}_{A}} = \frac{{H}^{2}}{D} \frac{Q}{HLW} = \frac{Q}{λDW}$ with *k_{A}* being the mass transport rate due to advection (here over distance *L*), *k_{D}* the mass transport rate due to diffusion (here over distance *H), τ_{D}* the characteristic diffusive time scale, *τ_{A}* the characteristic advective time scale, *H* the height of the measurement chamber, *D* the diffusion coefficient of an analyte, *Q* the flow rate, *W* the width of the measurement chamber, and *λ = L*/*H* the aspect ratio of the measurement chamber. From Equation S13 can be concluded that for *Pe_{L} >* 1 the *τ_{D}* is larger than *τ_{A}* and therefore the mass transport is diffusion-limited. For *Pe_{L} < 1, τ_{D}* is smaller than *τ_{A}* which causes the mass transport to be advection-limited.

### Supplementary information 6: the influence of time-controlled analyte exchange on the sensor performance

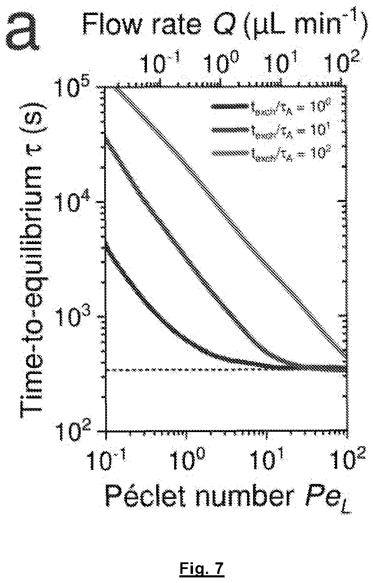

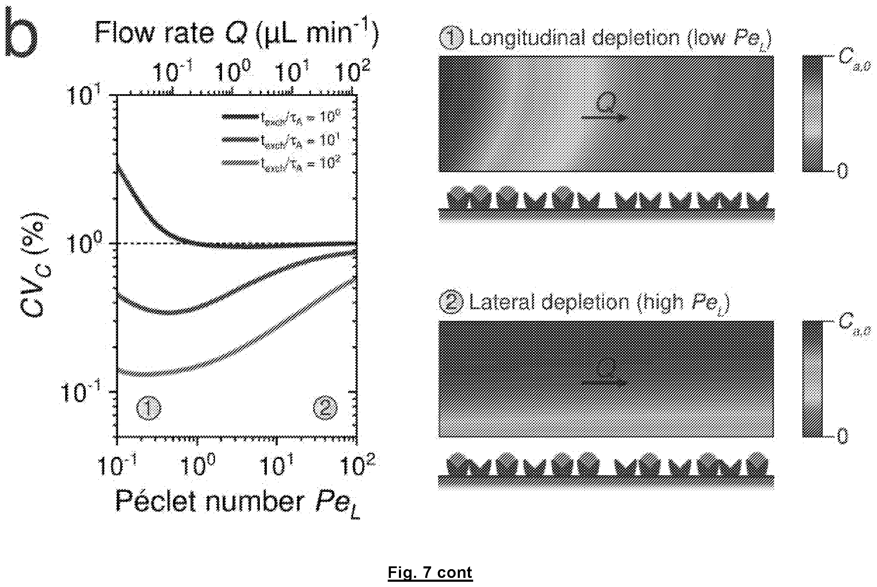

The influence of flow rate *Q* on the observed time-to-equilibrium *τ* and the precision of the concentration determination *CV_{C}* is quantified in Figure 7 for a sensor with standard parameter values as listed in Table 2 and a geometry as given in Figure 6A, top. Figure 7A shows the time-to-equilibrium *τ* as a function of longitudinal Péclet number *Pe_{L},* by varying the flow rate *Q*, for three values of *t*_{exch}/*τ*_{A} (see Supplementary Information 5). For small *Pe_{L}* the mass transport by advection is slow compared to mass transport by diffusion, and thus *τ* is advection-limited and scales according to *τ* ∝ 1/Q (see Note 7). Besides, increasing the duration of the primary exchange *t*_{exch} causes the observed time-to-equilibrium *τ* to be longer. The plateau value (dashed line) is reached when mass transport by advection is fast compared to mass transport by diffusion, *i.e.,* at high flow rates, which corresponds to the *τ* found in Figure 2 for an equal sensor height. In this regime, the sensor can be assumed to be incubated with a concentration *C*_{*a,*0} instantaneously. For *Pe_{L}* → ∞, a *τ* is observed which equals the found *τ* for instantaneous analyte exchange (see Figure 2A-Figure 2B).

| **Note 7. The influence of analyte exchange by advection on the time-to-equilibrium τ** |
|---|
| When the time-to-equilibrium is advection-limited, this results in τ = t_{exch}: |
| $τ = {t}_{exch} \to τ = \frac{HLW}{Q} \frac{{t}_{exch}}{{τ}_{A}} = \frac{λDW}{Q} \frac{HL}{λD} \frac{{t}_{exch}}{{τ}_{A}} = \frac{1}{{Pe}_{L}} \frac{{H}^{2}}{D} \frac{{t}_{exch}}{{τ}_{A}}$ |
| $τ ∝ \frac{1}{{Pe}_{L}} \to τ : {Pe}_{L} = 1 : 1 .$ |

Figure 7B shows the precision of the concentration determination as a function of the longitudinal Péclet number *Pe_{L}* and flow rate *Q* on the secondary x-axis. Again, roughly two regimes can be identified: for small *Pe_{L}*, *CV_{C}* depends on *Pe_{L}* since the analytes entering the measurement chamber bind to the binders on surface, which results in longitudinal depletion; this results in a positional dependency of the density *γ_{ab}* of analyte-binder complexes and thus the precision. For large *Pe_{L}*, the analyte exchange by advection is much faster than analyte exchange by diffusion causing almost instantaneous exchange of material, which results in lateral depletion, and thus an independency of the precision on *Pe_{L}.* For *Pe_{L}* → ∞, a *CV_{C}* is observed which equals the found *CV_{C}* for instantaneous analyte exchange (see Figure 5D-Figure 5E).

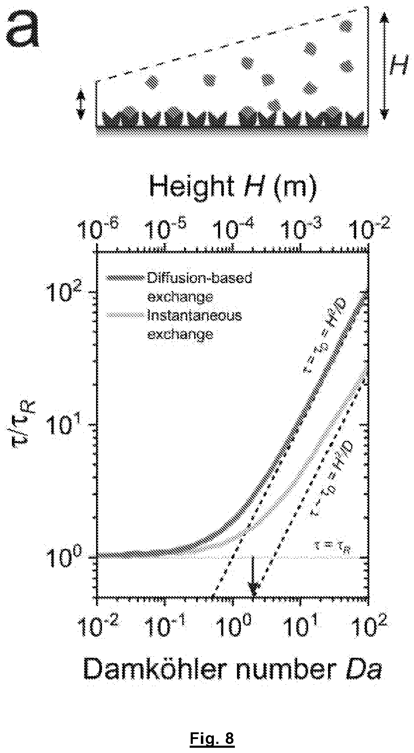

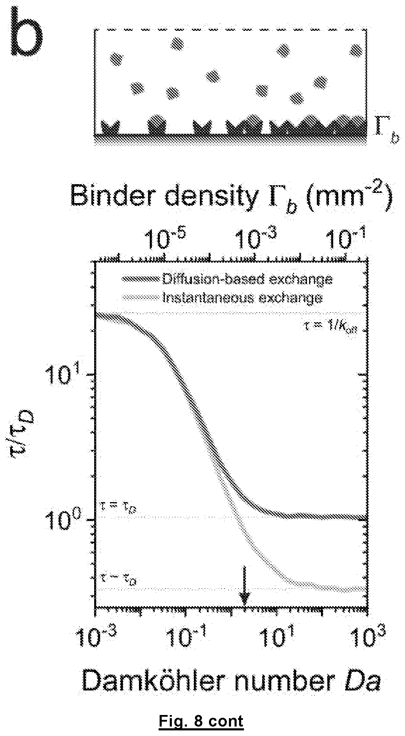

Figure 8 shows the influence of diffusion-based analyte exchange on the observed time-to-equilibrium, by comparing instantaneous analyte exchange (see Figure 2) to diffusion-based analyte exchange, where *t_{exch}*/*τ_{D}* = 1. Figure 8A shows the time-to-equilibrium *τ* as a function of Damköhler number *Da* for diffusion-based analyte exchange (dark orange) and instantaneous analyte exchange (light orange, same data as Figure 2A), by varying the height *H* of the measurement chamber. At low *Da* (*i.e*., at small measurement chamber height *H*), for both exchange methods, the observed time-to-equilibrium is reaction-limited since the diffusion time scale is fast compared to the reaction time scale. However, for increasing *Da,* the time-to-equilibrium increases faster for diffusion-based exchange since the characteristic length scale *L_{D}* over which the molecules have to diffuse is larger (*L_{D} = H*) compared to instantaneous analyte exchange (on average *L_{D}* ≅ *H*/2).

Figure 8B shows the time-to-equilibrium *τ* as a function of *Da* for diffusion-based analyte exchange (dark orange) and instantaneous analyte exchange (light orange, same data as Figure 2B), by varying the binder density Γ*_{b}*. At low *Da* (*i.e*., at low surface binder density Γ*_{b}*), for both exchange methods, the observed time-to-equilibrium is reaction-limited since the reaction time scale is slow (*τ_{R}* = 1/*k*_{off}) compared to the diffusion time scale. However, at high *Da* where the time-to-equilibrium *τ* is determined by the diffusion time scale, *τ* is larger for diffusion-based exchange since the characteristic length scale *L_{D}* over which the molecules have to diffuse is larger.

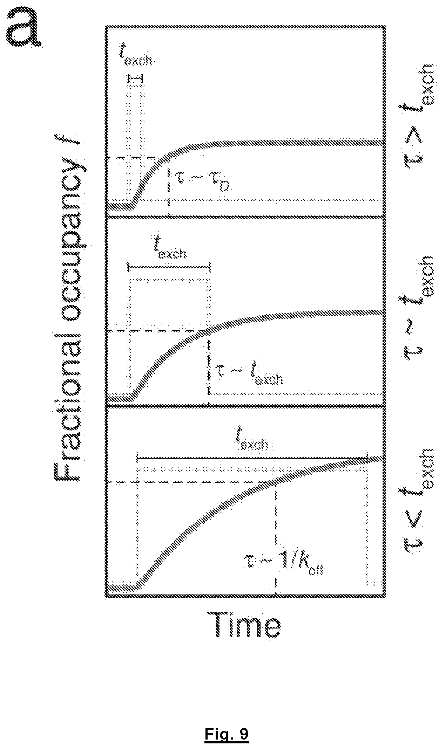

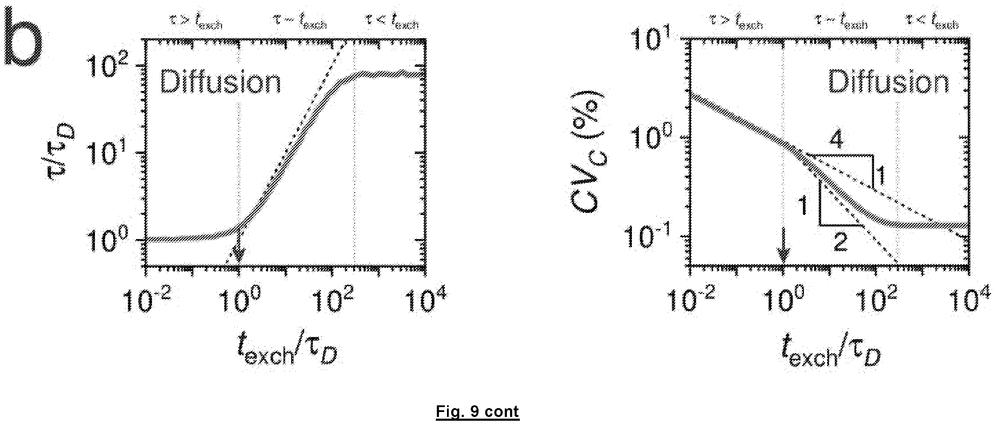

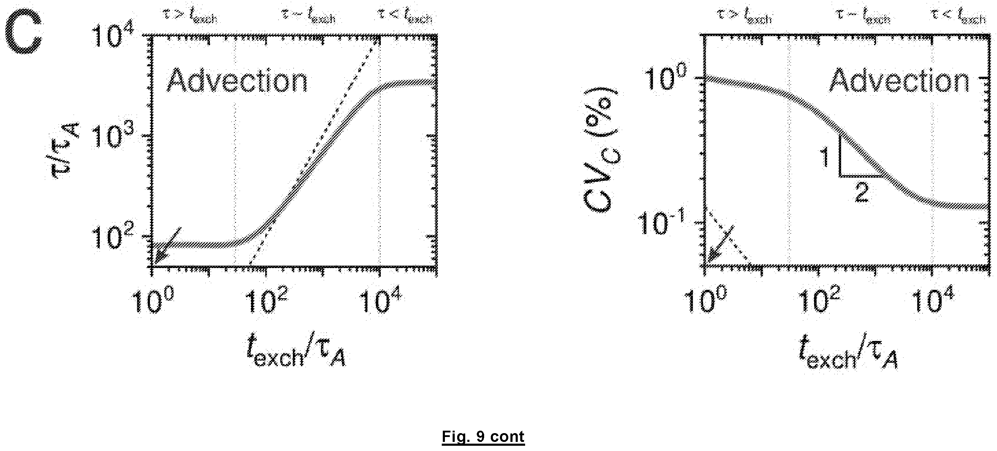

Figure 9 shows the influence of the primary exchange on the time-to-equilibrium and the sensitivity of the sensor for two monitoring geometries as presented in Figure 6 and using the standard parameter values given in Table 2. Figure 9A sketches the time-evolution of the fractional occupancy (solid orange line) and the primary exchange (dashed light orange line) by advection (by controlling flow rate *Q*) or diffusion (by controlling membrane permeability *P*). Three regimes are given here, where (1) *τ* is determined by the mass transport by diffusion within the measurement chamber, (2) *τ* is determined by the duration of the primary exchange *t_{exch},* and (3) *τ* is determined by the dissociation rate constant *k*_{off}. Note that for a short *t*_{exch}, the fractional occupancy when equilibrium is reached, is lower, since less molecules are exchanged between the measurement chamber and the system of interest.

Figure 9B shows the simulated results of the time-to-equilibrium *τ* normalized to the diffusion time scale *τ_{D}* as a function of the duration of the primary exchange *t*_{exch} normalized to the diffusion time scale *τ_{D}* (left) and the coefficient of variation of the concentration *CV_{C}* as a function of *t*_{exch}/*τ_{D}* (right). The three regimes as visualized in panel a could be observed in both graphs. For a small *t*_{exch}/*τ_{D}*, the observed time-to-equilibrium *τ* is diffusion-limited, since *t*_{exch} is much smaller than *τ_{D}*. However, *CV_{C}* is high (*i.e.,* precision is low) since the number of exchanged analytes is small; here, *CV_{C}* scales according to ${CV}_{C} ∝ 1 / \sqrt[4]{{t}_{exch} / {τ}_{D}}$ (see Note 8). For a large *t*_{exch}/*τ_{D}*, the observed time-to-equilibrium *τ* is reaction-limited, since the assay converts into an infinite-volume assay. However, *CV_{C}* is low (*i.e.,* precision is high) since the number of exchanged analytes is large (*i.e.,* there is an infinite supply of analytes). Here the precision is independent of the primary exchange, since the reaction reaches an equilibrium under infinite supply of analytes. When *τ* ~ *t*_{exch}, the time-to-equilibrium is mainly determined by the duration of the primary exchange since the assay can be regarded as neither a limited-volume assay nor an infinite-volume assay; here, *CV_{C}* scales roughly according to ${CV}_{C} ∝ 1 / \sqrt{{t}_{exch} / {τ}_{D}}$ (see Note 8).

| **Note 8. The mathematical dependency of sensitivity for analyte exchange by diffusion** |
|---|
| Fick's First Law gives ${J}_{a}^{D} = - D ∇ {C}_{a}$, where ${J}_{a}^{D}$ is the diffusion flux of analytes, which translates into ${J}_{a}^{D} = - D \frac{{δC}_{a}}{δy}$ assuming one-dimensional transversal diffusion. The maximum density ${γ}_{ab}^{max}$ of analyte-binder complexes that can be reached, is calculated by ${γ}_{ab}^{max} = {J}_{a}^{D} {t}_{exch}$. The mean length L_{D} over which analytes diffuse during t_{exch} equals $δy = {L}_{D} = \sqrt{{H}^{2} \frac{{t}_{exch}}{{τ}_{D}}}$ for L_{D} < H and δy = H for L_{D} ≥ H. |
| For L_{D} < H, gives ${γ}_{ab}^{max} = {HC}_{a} \sqrt{\frac{{t}_{exch}}{{τ}_{D}}}$ and for L_{D} ≥ H, gives ${γ}_{ab}^{max} = {HC}_{a} \frac{{t}_{exch}}{{τ}_{D}}$. The maximum achievable coefficient of variation can be calculated by ${CV}_{C} = \frac{1}{\sqrt{{γ}_{ab}^{max} {A}_{s}}}$, where Aₛ is the signal collection area. |
| For L_{D} < H, gives ${CV}_{C} ∝ \frac{1}{\sqrt[4]{\frac{{t}_{exch}}{{τ}_{D}}}}$ and for L_{D} ≥ H and ${CV}_{C} ∝ \frac{1}{\sqrt{\frac{{t}_{exch}}{{τ}_{D}}}}$. |

Figure 9C shows the simulated results of the time-to-equilibrium *τ*, normalized to the advection time scale *τ_{A}*, as a function of the duration of the primary exchange *t*_{exch}, normalized to the advection time scale *τ_{A}* (left), and the coefficient of variation of the concentration *CV_{C}* as a function of *t*_{exch}/*τ_{A}* (right). Again, the three regimes as visualized in panel a are observed in both graphs. The behavior is similar to panel b: for a small *t*_{exch}/*τ_{A}*, the observed time-to-equilibrium *τ* is diffusion-limited, but *CV_{C}* is high (*i.e*., precision is low) since the number of exchanged analytes is small. No values are shown for *t*_{exch}/*τ_{A}* < 1 due to the development of positional dependency of *γ_{ab}* when the volume of the measurement chamber is not fully exchanged (see Figure 7B). Besides, at low *t*_{exch}/*τ_{A}*, the precision is roughly independent of *t*_{exch}/*τ_{A}* due to the development of a lateral depletion zone (see Figure 7B). For a large *t*_{exch}/*τ_{A}*, the observed time-to-equilibrium *τ* is reaction-limited and *CV_{C}* is low (*i.e.,* precision is high) since the number of exchanged analytes is large (*i.e.,* there is an infinite supply of analytes). When *τ* ~ *t*_{exch}, the time-to-equilibrium is mainly determined by the duration of the primary exchange and *CV_{C}* scales roughly according to ${CV}_{C} ∝ 1 / \sqrt{{t}_{exch} / {τ}_{A}}$ (see Note 9). However this expected *CV_{C}* based on the number of analytes entering the measurement chamber (see black dashed line, left bottom corner), is lower than the observed *CV_{C}* (see also panel b). The difference between analyte exchange by diffusion and by advection, is that in the case of analyte exchange by advection, analytes can be lost through the oulet without contributing to the precision of the sensor. Therefore the maximum density ${γ}_{ab}^{max}$ can be reached in diffusion-based analyte exchange, while it cannot be reached in advection-based analyte exchange.

| **Note 9. The mathematical dependency of sensitivity for analyte exchange by advection** |
|---|
| The advective flux of analytes ${J}_{a}^{A}$ can be described by ${J}_{a}^{A} = {QC}_{a , 0}$. The maximum analyte-binder surface density ${γ}_{ab}^{max}$ can be calculated by ${γ}_{ab}^{max} = \frac{{J}_{a}^{A}}{WL} {t}_{exch} \to {γ}_{ab}^{max} =$ ${HC}_{a , 0} \frac{{t}_{exch}}{{τ}_{A}}$ , assuming *L_{A}* ≥ *L* with lateral depletion. The maximum achievable coefficient of variation can be calculated by ${CV}_{C} = \frac{1}{\sqrt{{γ}_{ab}^{max} {A}_{s}}}$, where Aₛ is the signal collection area. |
| For *L_{A}* ≥ *L,* gives ${CV}_{C} ∝ \frac{1}{\sqrt{\frac{{t}_{exch}}{{τ}_{A}}}}$. |
| $\to {CV}_{C} : \frac{{t}_{exch}}{{τ}_{A}} = 1 : 2$ for *l*_{adv} ≥ *L* |

### Supplementary Information 7: biosensing by Particle Mobility

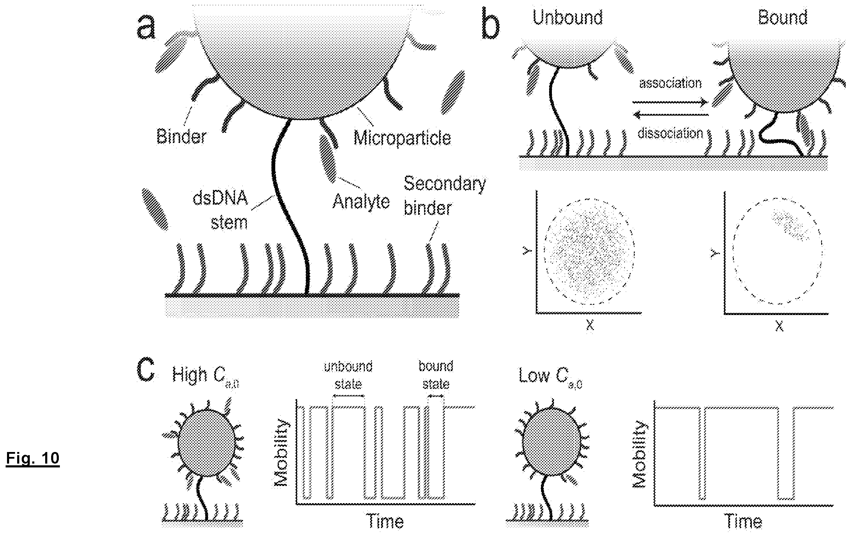

Here the concept of rapid monitoring of low-concentration analytes by time-controlled analyte exchange is experimentally demonstrated using Biosensing by Particle Mobility (BPM), a biosensing method with both single-particle and single-molecule resolution. The molecular design and measurement principle are sketched in Figure 10, illustrated with a sandwich assay format. Figure 10A shows a particle that is tethered to a substrate by a dsDNA tether and functionalized with ssDNA binder molecules, and a surface that is functionalized with secondary binder molecules.

Figure 10B illustrates the sensing functionality of the BPM system. The secondary binder molecules can transiently bind to analytes captured from solution by the binder molecules on the particle. The transient binding affects the mobility of the particle, because an unbound particle has a larger in-plane motional freedom than a bound particle. Two mobility time traces are sketched in Figure 10C, at a high (left) and low (right) analyte concentration. The switching frequency, *i.e.,* the activity, of the particle depends on the analyte concentration, because the unbound state lifetime of a particle decreases when the number of captured analytes increases.

In order to demonstrate the rapid monitoring methodology for low-concentration analytes using BPM, ssDNA analytes were used that bind with a 20nt interaction to the ssDNA binder molecules on the particle. The particles are functionalized with a high binder density and have a high-affinity interaction with the analyte (characteristic lifetime of several hours), which implies that *C_{b,ch} > C*_{*a,*0} and *C_{b,ch} > K_{d}*, and therefore the effective volumetric binder concentration dominates the time-to-equilibrium of the reaction.

### Supplementary Information 8: precision of biosensing by Particle Mobility with time-controlled analyte exchange

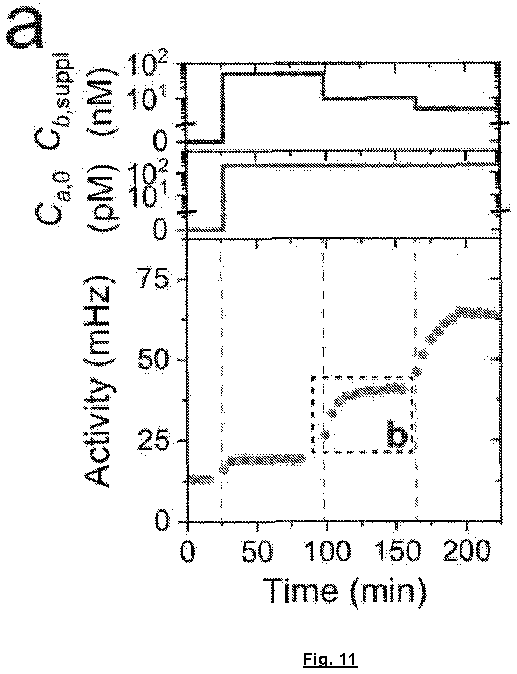

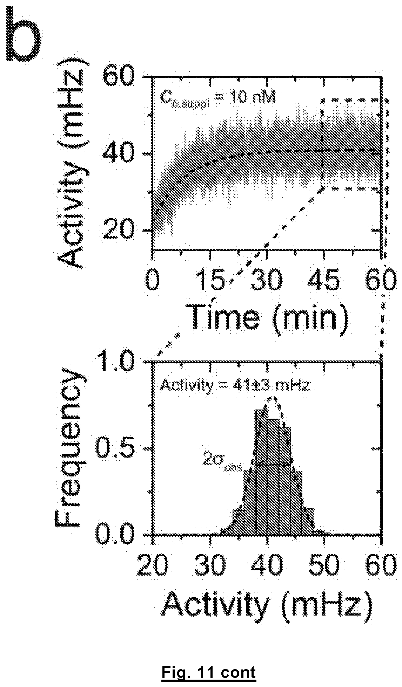

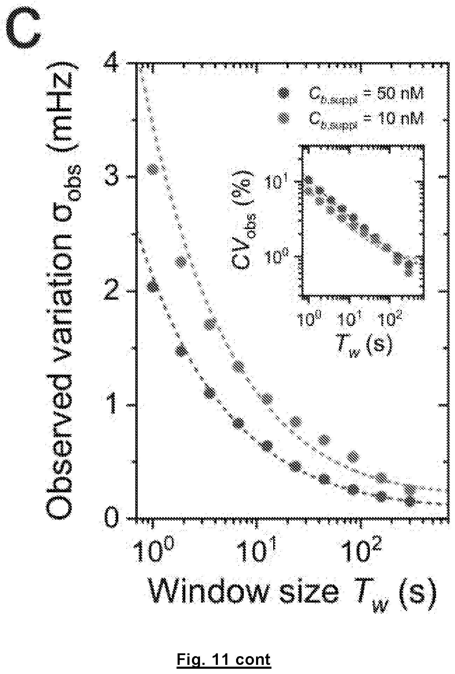

The results of BPM measurements with time-controlled analyte exchange are given in Figure 11. Figure 11A shows the measured activity per measurement block of 5 minutes as a function of time for multiple consecutive measurement cycles (bottom). At the start of each cycle (see vertical grey lines), the measurement chamber was filled with a solution containing input analyte concentration *C*_{*a,*0} = 200 pM (middle) and a varying supplemented binder concentration *C*_{*b*,suppl} (top). The data show that the time-to-equilibrium is shorter in a condition with high supplemented binder concentration. The data in Figure 11A were fitted in order to extract values for the time-to-equilibrium *τ* and for the signal change Δ*S*; the fitted values for *τ* and Δ*S* are plotted in Figure 4B and discussed in the body text of the description of the present invention.

It is questioned to what extent the precision of the BPM sensor is limited by Poisson statistics (*cf*. Figure 5). The total variation observed in a measurement is quantified and the variation induced by the measurement itself is calculated. Using this approach the variation caused by other sources than the measurement can be estimated and compared to the variation caused by the discrete number of analyte-binder complexes on the particles, *i.e*., the Poisson-limited variation.

Figure 11B shows a zoom-in of the measurement cycle where *C*_{*b*,suppl} = 10 nM (top) and the distribution of the observed activity when the reaction is in equilibrium (bottom). The activity as a function of time is shown with a moving average, with a time window *T_{w}* of 1 s, of which the observed variation of the activity equals *σ*_{obs} = 3 mHz. The activity as a function of time is fitted by a single exponential of the form given in Note 3 (top, dashed line), from which the time-to-equilibrium *τ* and Δ*S* were extracted (see Figure 4B). The distribution of the observed activity is fitted with a normal distribution from which the mean activity *µ_{A}* and *σ*_{obs} are extracted.

Figure 11C shows *σ*_{obs} as a function of *T_{w}* for the first cycle (*C*_{*b*,suppl} = 50 nM, brown) and the second cycle (*C*_{*b*,suppl} = 10 nM, orange). Increasing the time window *T_{w},* results in a smaller *σ*_{obs} since the calculated activity is averaged over more time points. The observed variation in the activity depends on the variation induced by the measurement and by other sources of variation: ${σ}_{obs}^{2} = {σ}_{meas}^{2} + {σ}_{other}^{2}$ where ${σ}_{meas} = {σ}_{ref} / \sqrt{{T}_{w}}$, with *σ*ₘₑₐₛ the measurement induced variation, *σ*_{ref} a reference variation which is taken as the measurement induced variation at *T_{w} =* 1 s, *T_{w}* the time window of the moving average of the activity as a function of time, and *σ*ₒₜₕₑᵣ the variation from a different source than the measurement itself, *e.g*., a discrete number of analyte-binder complexes or variations in surface chemistry. If the precision of a sensor is Poisson-limited, *σ*ₒₜₕₑᵣ equals *σ*_{Poisson}, where *σ*_{Poisson} is the variation caused by the discrete number of observed analyte-binder complexes within the signal collection area.

For a BPM measurement, the number of observed analyte-binder complexes can be calculated by: ${N}_{ab}^{obs} = {γ}_{ab}^{eff} {A}_{s} {ϵ}_{p}^{obs}$
where ${γ}_{ab}^{eff}$ is the effective analyte-binder complex density, *Aₛ* the signal collection area and ${ϵ}_{p}^{obs}$ the observed fraction of the particle area. In a BPM measurement with 1 µm particles, only approximately 2% of the particle surface is contributes to the observed signal¹, which results in ${ϵ}_{p}^{obs} = 0.02$. ${γ}_{ab}^{eff}$ can be calculated by: ${γ}_{ab}^{eff} = {f}_{a}^{eff} {f}_{end} {Γ}_{b}$
where ${f}_{a}^{eff} = {Γ}_{b} / \left({HC}_{b , suppl}+{Γ}_{b}\right)$ is the effective fraction of the total analytes captured by the binders on the surface, and *f*_{end} the fractional occupancy of all binder molecules by analytes at the end of a measurement cycle for a given input analyte concentration *C*_{*a*,0}. Note that ${f}_{a}^{eff}$ can be larger than 1 when multiple consecutive cycles have been measured: for the first cycle, ${f}_{a}^{eff} = {Γ}_{b} / \left({HC}_{b , suppl , 1}+{Γ}_{b}\right)$, while for the second cycle, ${f}_{a}^{eff} = {Γ}_{b} / \left({HC}_{b , suppl , 1}+{Γ}_{b}\right) + {Γ}_{b} / \left({HC}_{b , suppl , 2}+{Γ}_{b}\right)$. Using standard parameter values from Table 2, *f*_{end} = 10⁻² (extrapolated from Figure 5A at *C*_{*a,*0} = 200 pM), and a signal collection area of *Aₛ* = 1 mm², it can be found that ${σ}_{Poisson}^{∗} = \sqrt{{N}_{ab}^{obs}} = 1.0 ⋅ {10}^{2}$ for the first cycle (where *C*_{*b*,suppl} = 50 nM) and that ${σ}_{Poisson}^{∗} = 2.3 ⋅ {10}^{2}$ for the second cycle (where *C*_{*b,*suppl} = 10 nM). Assuming a Poisson-limited sensor, the variation in the observed activity equals *σ*_{Poisson} *= CV*_{Poisson} *µ_{A}* where ${CV}_{Poisson} = {σ}_{Poisson}^{∗} / {N}_{ab}^{obs}$ is the coefficient of variation in the observed number of analyte-binder complexes and *µ_{A}* the mean observed activity at equilibrium (see panel a). This gives *CV*_{Poisson} = 9.6 · 10⁻³ and therefore *σ*_{Poisson} = 0.18 mHz for the first incubation cycle (where *C*_{*b*,suppl} *=* 50 nM), and *CV*_{Poisson} *=* 4.4 · 10⁻³ and therefore *σ*_{Poisson} *=* 0.18 mHz for the second incubation cycle (where *C*_{*b*,suppl} *=* 10 nM).

The dashed lines in Figure 11C represents the fit according to Equation S14. By taking the limit *T_{w}* → ∞, *σ*_{obs} equals *σ*ₒₜₕₑᵣ. At the first cycle with *C*_{*b*,suppl} = 50 nM, *σ*ₒₜₕₑᵣ was found to be equal to *σ*ₒₜₕₑᵣ = 0.09 ± 0.02 mHz, while at the second cycle with *C*_{*b*,suppl} = 10 nM, *σ*ₒₜₕₑᵣ = 0.21 ± 0.03 mHz. Comparing these values to the previously quantified *σ*_{Poisson}, one can conclude that σₒₜₕₑᵣ ≅ *σ*_{Poisson}, which indicates that the precision in the BPM measurement is Poisson-limited. Therefore, the precision of the BPM measurements are determined by the fundamental limit of stochastic fluctuations in the number of analyte-binder complexes, and can be compared to the results given in Figure 5C-Figure 5D.

### Brief description of the drawings

The following section briefly discusses the different drawings.

### Figure 1: concept of the sensing methodology for the rapid monitoring of low analyte concentrations

Figure 1A. Sensing system for analyte monitoring. Analytes are exchanged between a biological or biotechnological system of interest and a measurement chamber. The data result in a concentration-time profile which should correspond as close as possible to the true analyte concentration in the system of interest. Figure 1B. Geometry of the measurement chamber, with height *H,* width *W,* and length *L.* A reaction rate at the sensor surface is caused by the association and dissociation between analytes (orange) and binder molecules (brown), described by the association rate constant *kₒₙ,* the dissociation rate constant *k_{off},* the total binder density Γ*_{b}*, the analyte concentration *Cₐ* near the surface, and the density of analyte-binder complexes *γ_{ab}*. Analyte exchange is facilitated by diffusion and advection, where diffusion occurs in both x- and y-direction with diffusion coefficient *D*, resulting in a net molar flux *Jₐ*, and where advection occurs in the x-direction only, with a developed flow profile with flow rate *Q* and a mean flow speed *vₘ*. Figure 1C. The time profile of the sensor response for low analyte concentration (*C*_{*a,*0} < *K_{d}*), for two conditions: infinite-volume and limited-volume assays. Measuring in an infinite volume results in an excess of analytes compared to binder molecules (*C*_{*a,*0} *> C_{b,ch}*), causing the time-to-equilibrium *τ* to be determined by *k*_{off}. The limited-volume condition is defined as a condition where binder molecules are in excess compared to analytes (*C*_{*b*,*ch*} > *C*_{*a*,0}, with *C_{b,ch} =* Γ*_{b}*/*H*) and in excess compared to the equilibrium dissociation constant (*C_{b,ch} > K_{d}*). This causes *τ* to be determined by the effective binder concentration *(i.e.,* measurement chamber height *H* and binder density Γ*_{b}*), which is much shorter than 1/k_{off}. Figure 1D. Analyte monitoring using a limited-volume assay involves repeated cycles with two phases. In phase 1, the primary exchange phase, analytes are exchanged effectively between the system of interest and the measurement chamber. In phase 2, the secondary exchange phase, analytes are exchanged ineffectively. Recording the time-dependent signal during the secondary exchange phase (in the middle of the measurement chamber at distance *L*/2 from the entrance), reveals the analyte concentration. Inside the measurement chamber, the limited-volume condition gives a time-dependence of the analyte concentration: a decrease over time (depletion) or an increase over time (repletion), depending on the input analyte concentration *C*_{*a,*0} and the initial occupation *f*ᵢₙᵢₜ of binders by analytes. The input analyte concentration *C*_{*a,*0} is derived from the measured time-dependence of the fractional occupation *f*(*t*).

### Figure 2: time-to-equilibrium τ of a limited-volume assay for a sensor design with different heights, binder densities, and flow rates of analyte exchange

Figure 2A. Time-to-equilibrium *τ* as a function of measurement chamber height *H* (orange line) for an instantaneous analyte exchange. For small *H,* the observed *τ* is reaction-dominated (*τ = τ_{R} =* 1/(*τ_{R,LV}*⁻¹ *+ k_{off}*), black dotted line), while for increasing *H* the observed *τ* becomes diffusion-dominated. The inset shows the same data, where *τ* is normalized to *τ_{R}* and plotted as a function of Damköhler number *Da.* The sketch above the graph visualizes a measurement chamber with an increasing measurement chamber height. Figure 2B. Time-to-equilibrium *τ* as a function of the binder density *Γ_{b}* (orange line) for an instantaneous analyte exchange. For low Γ*_{b}*, the observed *τ* is reaction-dominated (*τ = τ_{R}*, black dotted line), while for increasing *Γ_{b}* the observed *τ* becomes diffusion-dominated. The inset shows the same data, where *τ* is normalized to the characteristic diffusion time *τ_{D}* and plotted as a function of *Da.* For low *Da*, *τ* is limited by *1*/*k_{off},* while at high *Da*, *τ* is limited by *τ_{D}*. The sketch above the graph visualizes a measurement chamber with an increasing binder density. Figure 2C. Time-to-equilibrium *τ* as a function of flow rate *Q* for three aspect ratios *λ = L*/*H*, for time-controlled analyte exchange by advection where the primary exchange phase duration *t_{pr.exch}* equals the characteristic advection time *τ_{A}*. For small *Q*, the observed *τ* is limited by the advective transport of analytes from the inlet toward the point of sensing at distance *L*/2 from the inlet. For increasing *Q*, this transport process becomes faster causing the observed *τ* to be dominated by reaction and/or diffusion at high flow rates. The inset shows the same data (*Da =* 2) supplemented with *Da =* 0.2 (reaction-limited) and *Da =* 20 (diffusion-limited), where *τ* is normalized to *τ_{R}* and plotted as a function of the longitudinal Péclet number *Pe_{L}.* The dotted lines show the *τ*/*τ_{R}* value at high *Q* and are comparable to the values found in panel a. The sketch above the graph visualizes a measurement chamber with an increasing flow rate. In all panels, the black arrows on the x-axis indicate the standard parameter values for *H*, Γ*_{b}*, and *Q* as listed in Table 1.

### Figure 3: Simulated response of the analyte monitoring system using time-controlled analyte exchange

Figure 3A. The analyte concentration *Cₐ* in the measurement chamber (brown line) and the fractional occupancy *f* of binder molecules by analytes (orange line) as a function of time, for low *f*ᵢₙᵢₜ and depletion of analyte in solution (left), and for high *f*ᵢₙᵢₜ and repletion of analyte in solution (right). The dashed lines indicate time points where instantaneous analyte exchange occurs, where the bulk analyte concentration was set to *C*_{*a,*0} = 0.1 pM after a period of approximately 50 min. The insets highlight the kinetics of the first cycles, showing a time-to-equilibrium of *τ* = 340 s. For many cycles (*n* → ∞) both curves would approach ${f}_{eq , IV} = \frac{{C}_{a , 0}}{{C}_{a , 0} + {K}_{d}} = 10 ⋅ {10}^{- 4}$, which equals the equilibrium value when an infinite volume is supplied (see Table 2). Figure 3B. The fractional occupancy *f* as a function of time where cycles of analyte exchange and incubation are applied every 15 min with alternatingly *C*_{*a,*0} = 0.15 pM and *C*_{*a,*0} = 0.05 pM. The curve saturates at *f_{eq.IV} =* 10 · 10⁻⁴, which equals the infinite-volume equilibrium value for the average concentration value *C*_{*a,*0} = 0.1 pM. Dashed lines: continuous supply of *C*_{*a,*0} = 0.05 pM yields *f_{eq,IV}* = 5 · 10⁻⁴ and *C*_{*a,*0} = 0.15 pM yields *f_{eq,IV} =* 15 · 10⁻⁴. The right panel shows zoom-ins of three sections of the solid curve, each representing four cycles of instantaneous analyte exchange and subsequent incubations of 15 minutes. In zoom-in 1 (*t* = 0 - 1 h) all curve segments show depletion behavior. In zoom-ins 2 (t = 12 - 13 h) and 3 (*t* = 42 - 43 h), depletion is seen for *C*_{*a,*0} = 0.15 pM, since *f*ᵢₙᵢₜ *< f_{eq,IV}*(*C*_{*a,*0} = 0.15 pM), and repletion is seen for *C*_{*a,*0} = 0.05 pM, since *f*ᵢₙᵢₜ *> f_{eq,IV}*(*C*_{*a,*0} *=* 0.05 pM). For all curve segments, the time-to-equilibrium *τ* = 340 s. The vertical scale bars indicate Δ*f =* 10⁻⁴.

### Figure 4: experimental study of a limited-volume assay with varying supplemented binder concentrations using Biosensing by Particle Mobility (BPM)

Figure 4A. Sketch of the measurement chamber in a BPM measurement (see Supplementary Information 7) without (top) and with (bottom) supplemented binders with concentration *C*_{*b,*suppl}. For simplicity the particles of the BPM sensor are not shown in the sketch. In the absence of supplemented binders, the total binder concentration *C*_{*b,*tot} equals *C*_{*b,*tot} *=* Γ*_{b}*/*H*; in the presence of supplemented binders, the total binder concentration *C*_{*b,*tot} equals *C*_{*b,*tot} *=* Γ*_{b}*/*H + C*_{*b*,suppl}. Supplemented binders give a shorter time-to-equilibrium since the time-to-equilibrium scales according to *τ_{R,LV}* ∝ 1/*C*_{*b,*tot} (see Table 1). Supplemented binders give a lower signal change because analytes captured in solution do not generate signal on the sensor surface. Figure 4B. Experimentally observed time-to-equilibrium *τ* (left) and normalized signal change Δ*S* (right) as a function of supplemented binder concentration *C*_{*b*,suppl} in a BPM measurement with DNA-DNA hybridization reaction for an analyte concentration of 200 pM (see Supplementary Information 7). Left: the dashed line shows the fitted curve *τ* = *p*₁/(*p*₂ + *C*_{*b,*suppl}) + *p*₃, where *p₁ =* 1/*k*ₒₙ *(kₒₙ* is assumed to be equal for all binders), *p₂ =* Γ*_{b}*/*H*, and *p*₃ is the delay contributed by diffusion (see *τ_{D}*, black line, see also Figure 2B) and experimental steps. Assuming *H* = 200 µm (see Table 1), the fit gives Γ*_{b}* = (3 ± 1) · 10⁻¹⁰ mol m⁻², which is comparable to the standard parameter value as listed in Table 1. The fitted association rate constant is *k*ₒₙ *=* (1.5 ± 0.4) · 10⁵ M⁻¹ s⁻¹, which is in the range of values reported in literature for comparable DNA-DNA hybridization reactions. Right: in the depletion condition (*f*ᵢₙᵢₜ *< f_{eq,IV}*) the fractional occupancy scales according to *f* ∝ 1/*C*_{*b,*tot} = *C*_{*a,*0}/(*C*_{*b*,*ch*} + *C*_{*b*,suppl}). The dashed line shows the fitted curve Δ*S = p*₁/(*p*₂ *+ C*_{*b*,suppl}), where *p*₁ scales the change in fractional occupancy to signal change and *p*₂ = Γ*_{b}*/*H.* For *H* = 200 µm, it was found that Γ*_{b}* = (7 ± 4) · 10⁻¹⁰ mol m⁻², which is comparable to the previously found value for Γ*_{b}* and the standard parameter value as listed in Table 1. The insets show the same data on lin-log scales. The errors reported in the Figure (smaller than the symbol size) and the caption are fitting errors based on a 68% confidence interval.

### Figure 5: analytical performance of the limited-volume assay, derived from simulations of a single measurement cycle

Figure 5A. Fractional occupancy at the end of the incubation *f_{end}* as a function of analyte concentration *C*_{*a*,0} for different initial fractional occupancies *fᵢₙᵢₜ.* The right y-axis indicates the number of surface-bound analytes at the end of the cycle ${γ}_{ab}^{end}$*.* Figure 5B. The absolute change of fractional occupancy *Δf* as a function of *C*_{*a*,0} for various *fᵢₙᵢₜ.* The right y-axis indicates *Δγ_{ab}.* A positive *Δf* and *Δγ_{ab}* indicate depletion; negative values indicate repletion. The inset shows the same data on a lin-lin scale. Figure 5C. The coefficient of variation *CV_{C}* with which the analyte concentration *C*_{*a*,0} can be determined as a function of analyte concentration *C*_{*a*,0} for various initial fractional occupancies *fᵢₙᵢₜ. CV_{C}* scales as $1 / \sqrt{{C}_{a , 0}}$ for low *fᵢₙᵢₜ* and high *C_{a,0}*; *CV_{C}* scales as 1/*C*_{*a*,0} for high *fᵢₙᵢₜ* and low *C*_{*a*,0}. Figure 5D. *CV_{C}* as a function of measurement chamber height *H* (top) and binder density *Γ_{b}* (bottom) for various initial fractional occupancies *fᵢₙᵢₜ* and *C*_{*a*,0} = 0.1 pM. The arrows on the x-axes indicate the standard parameter values for *H and Γ_{b}* which as listed in Table 1. Figure 5E. *CV_{C}* as a function of the observed time-to-equilibrium *τ* when varying the measurement chamber height *H* (left) or binder density *Γ_{b}* (right) for various initial fractional occupancies *fᵢₙᵢₜ* and *C*_{*a*,0} = 0.1 pM. The sketches above the graphs visualize a measurement chamber with an increasing height or a decreasing binder density. The arrows on the x-axes indicate the obtained time-to-equilibrium using the standard parameter values for *H and Γ_{b}* as listed in Table 1.

### Figure 6: time-controlled analyte exchange by advection and diffusion

Figure 6A. Concepts of analyte exchange by advection (top) and by diffusion (bottom) between a system of interest and a measurement chamber, using flow rate *Q* and molar flux *Jₐ* through a semi-permeable membrane respectively. Figure 6B. Schematic visualizations of time-controlled analyte exchange by controlling the flow rate *Q* (top) and the molar flux *Jₐ* (by controlling the membrane permeability *P*, bottom) in time. The primary exchange has a characteristic time *τ_{exch},* which equals τ*_{A}* for advection-based analyte exchange, and *τ_{D}* for diffusion-based analyte exchange, and a duration of *t_{exch}* in which analytes travel characteristic length *L_{A}* or *L_{D}.* Three regimes are identified: 1) *t_{exch} < τ_{exch}* where *L_{A} < L* or *L_{D} < H,* 2) *t_{exch} = τ_{exch}* where *L_{A} = L* or *L_{D} = H,* and 3) *t_{exch} > τ_{exch}* where *L_{A} > L or L_{D} > H.*

### Figure 7: the influence of advection-based analyte exchange on the performance of the analyte monitoring system using time-controlled analyte exchange by advection

Figure 7A. The time-to-equilibrium τ as a function of the longitudinal Péclet number *Pe_{L},* with the flow rate *Q* on the secondary x-axis (see Table 2), for three values of *t_{exch}*/*τ_{A}.* For small *Pe_{L},* τ depends on *Pe_{L}* where τ ∝ 1/*Pe_{L}* (see Note 7). τ decreases for increasing *Pe_{L}* since the primary exchange is faster (higher flow rate Q), but τ increases for increasing *t_{exch}*/*τ_{A}* since the duration of the primary exchange is longer. For large *Pe_{L},* τ is independent of *Pe_{L};* the *Pe_{L}* value where τ becomes independent of *Pe_{L},* depends on the *t_{exch}*/*τ_{A}.* Figure 7B. The precision of the measured concentration *CV_{C}* as a function of the longitudinal Péclet number *Pe_{L}* at an analyte concentration *C*_{*a*,0} = 0.1 pM, with the flow rate Q on the secondary x-axis (see Table 2), for three values of *t_{exch}*/τ*_{A}.* The schematic visualizations of the measurement chamber cross-section show the spatial distribution of the concentration *Cₐ*, where red equals high *Cₐ* (*Cₐ = C*_{*a,*0}) and blue equals low *Cₐ* (*Cₐ =* 0). For small *Pe_{L},* a longitudinal depletion zone appears where (almost) all analytes are captured from solution by binder molecules directly after entering the measurement chamber, causing a positional dependency of the density of analyte-binder complexes, and a lower *CV_{C}* (*i.e.,* a higher precision) at the point of sensing. For *t_{exch}*/*τ_{A} =* 10° this effect is largest since analyte exchange has a short duration. By increasing *t_{exch}*/τ*_{A},* the *CV_{C}* decreases since more analytes are exchanged. For large *Pe_{L},* a lateral depletion zone appears where the analyte exchange can be assumed to be instantaneous (see Figure 2); here *CV_{C}* is independent of *Pe_{L}* and no positional dependency of the density of analyte-binder complexes exists.

### Figure 8: influence of diffusion-based analyte exchange on the observed time-to-equilibrium τ

Figure 8A. Time-to-equilibrium τ normalized to the characteristic reaction time *τ_{R}* as a function of Damköhler number *Da,* with the measurement chamber height *H* on the secondary axis, for diffusion-based (orange) and instantaneous analyte exchange (light orange). For small *Da,* no difference in *τ*/*τ_{R}* exists since the observed reaction is reactionlimited. For large *Da,* diffusion-based analyte exchange results in a slower observed reaction since the characteristic distance *L_{D}* is larger. Figure 8B. The time-to-equilibrium τ normalized to the characteristic diffusion time *τ_{D}* as a function of Damköhler number *Da,* with the binder density *Γ_{b}* on the secondary axis. A difference in *τ*/*τ_{D}* exists caused by a longer *L_{D}.* For large *Da,* the observed reaction is diffusion-limited. In these simulations is was assumed that *t_{exch}*/τ*_{D} =* 1. In both panels, the black arrows on the x-axis indicate the standard parameter value for *Da* (using *H and Γ_{b}*) which is given in Table 2.

### Figure 9: the influence of the primary exchange on the performance of the analyte monitoring system using time-controlled analyte exchange

Figure 9A. Sketches of the time-evolution of the fractional occupancy upon analyte exchange by advection (by controlling flow rate *Q*) or diffusion (by controlling membrane permeability *P*). Three regimes are identified: 1) *τ > t_{exch}* where τ is determined by the mass transport by diffusion within the measurement chamber itself, 2) *τ ~ τ_{exch}* where τ is determined by the duration of the primary exchange, and 3) τ *< τ_{exch}* where τ is determined by the dissociation rate constant *k_{off}.* Figure 9B. Performance using time-controlled analyte exchange by diffusion for a sensor with parameters described in Table 2. Left: *τ*/*τ_{D}* as a function of *t_{exch}*/*τ_{D}.* For small *t_{exch}*/*τ_{D}* (where *τ > t_{exch}*), *τ* is independent of the exchange time since τ is limited by the mass transport after analyte exchange within the measurement chamber (reaction and diffusion, see Figure 2A-Figure 2B and Figure 8). For large *t_{exch}*/*τ_{D}* (where τ < *t_{exch}*), τ is independent of the exchange time since the assay can be considered as an infinite-volume assay. When *τ ~ t_{exch}, τ* is strongly determined by the duration of the primary exchange *t_{exch}.* The dashed black line represents τ *= t_{exch}.* Right: *CV_{C}* as a function of *t_{exch}*/*τ_{D}.* For small *t_{exch}*/*τ_{D}* (where τ> *t_{exch}*), *CV_{C}* depends on the amount of exchanged analytes where an increasing *t_{exch}* results in a decreasing *CV_{C}* where ${CV}_{C} ∝ \sqrt[4]{{t}_{exch}}$ (dashed black line, see Note 8). For large *t_{exch}*/*τ_{D}* (where τ < *t_{exch}*), *CV_{C}* is independent of *t_{exch}* since the assay can be considered as an infinite-volume assay. When *τ ~ t_{exch}, CV_{C}* depends more strongly on *t_{exch}* due to an increased molar flux *Jₐ*, where ${CV}_{C} ∝ \sqrt{{t}_{exch}}$ (dashed black line, see Note 8). The black arrows on the x-axis indicate the value for *t_{exch}*/*τ_{D}* = 1 used in Figure 8. Figure 9C. Performance using time-controlled analyte exchange by advection with *τ*/*τ_{A}* and *CV_{C}* as a function of *t_{exch}*/τ*_{A}.* Left: *τ*/*τ_{A}* as a function of *t_{exch}*/τ*_{A}.* The shape of the graph is similar to panel b, left, though shifted to higher values of *t_{exch}*/*τ_{A}* which depends on the flow rate which is used for analyte exchange. Besides, small *t_{exch}*/*τ_{A}* yield *τ*/*τ_{A}* ≠ 1 due to an additional diffusion time penalty caused by the mass transport within the measurement chamber, during and after the primary exchange. The dashed black line represents *τ* = *t_{exch}.* Right: For small *t_{exch}*/*τ_{A}* (where τ > *t_{exch}*), *CV_{C}* is roughly independent of *t_{exch}* since the analyte exchange by advection includes an outlet where analytes are lost, in contrast to analyte exchange by diffusion. Therefore the minimum *CV_{C}* which can be reached theoretically by analyte exchange by advection (dashed black line, see Note 9) is much lower than the observed *CV_{C}.* For large *t_{exch}*/*τ_{A}* (where τ < *t_{exch}*), *CV_{C}* is independent of *t_{exch}* since the assay can be considered as an infinite-volume assay. No values for *t_{exch}*/*τ_{A} <* 0 are shown for analyte exchange by advection, since in this regime positional dependency strongly influences τ and *CV_{C}.* The black arrows on the x-axis indicate the value for *t_{exch}*/*τ_{A} =* 1 used in Figure 7 and Figure 2C.

### Figure 10: measurement principle of Biosensing by Particle Mobility (BPM)

Figure 10A. Micrometer-sized particles (yellow) are tethered to a substrate using a dsDNA stem (black). The particle is functionalized with ssDNA binder molecules (brown) and the planar surface with ssDNA secondary binder molecules (light brown). Both binders can bind reversibly to single ssDNA analytes (orange) present in solution. Figure 10B. Analytes binding to the binder molecules on the particle and subsequently the secondary binder molecules on the planar surface cause the particle to exhibit distinct Brownian motion patterns, *i.e.,* the projection of the center of the particle onto the xyplane, corresponding to an unbound state (high mobility) or a bound state (low mobility). Figure 10C. Digital binding and unbinding events are identified by following the mobility of the particles over time. The time between two events corresponds to either the unbound state lifetime, or the bound state lifetime. For a high or low target concentration in solution, the microparticle shows a high or a low switching frequency respectively.

### Figure 11: the precision of Biosensing by Particle Mobility (BPM) measurements with time-controlled analyte exchange

Figure 11A. The response of a BPM sensor with time-controlled analyte exchange. Activity per measurement of 5 minutes as a function of time for multiple consecutive measurement cycles (orange). At the start of each cycle (vertical lines), *C*_{*a*,0} was set to 200 pM (light brown) and a varying supplemented binder concentration *C_{b,suppl}* was added (dark brown). Figure 11B. Top: zoom-in of the activity as a function of time calculated by a moving average with time window *T_{w} =* 1 s, for *C_{b,suppl} =* 10 nM (see panel a). The dashed line shows a single exponential fit (*Sᵢₙᵢₜ* +*ΔS · e*^{*-t*/}*^{τ},* see Supplementary Information 2), from which the time-to-equilibrium τ = 474 ± 2 s and the signal change Δ*S* = 19.02 ± 0.05 mHz could be determined (see Figure 4B). Bottom: distribution of the observed activity at equilibrium (*t* > 45 min). The dashed line is a fitted normal distribution with a mean activity µ*_{A}* and an observed variation *σ_{obs}.* Figure 11C. Observed variation *σ_{obs}* as a function of *T_{w}* for the first cycle (*C_{b,suppl}* = 50 nM, brown) and second cycle (*C_{b,suppl}* = 10 nM, orange). The dashed lines give the fit of the data according to Equation S14. For *T_{w}* → ∞, the observed variation *σ_{obs}* approaches the variation induced by other sources than the measurement itself *σₒₜₕₑᵣ,* which equals *σₒₜₕₑᵣ* = 0.09 ± 0.02 mHz for *C_{b,suppl}* = 50 nM and *σ_{obs}* = 0.21 ± 0.03 mHz for *C_{b,suppl} =* 10 nM. The inset shows the same data, with the coefficient of variation *CV_{obs}* as a function of *T_{w}.* For *T_{w}* → ∞, it was found that *CV_{obs} =* (7 ± 2) · 10⁻³ for *C_{b,suppl}* = 50 nM and *CV_{obs}* = (9 ± 1) · 10⁻³ for *C_{b,suppl} =* 10 nM. The errors reported in panel a are stochastic errors (smaller than the symbol size) while in panel c (smaller than the symbol size) and the caption of panel c, the reported errors are fitting errors based on a 68% confidence interval.

## Claims

1. Method for the continuous monitoring or intermittent testing of an analyte of interest, such as a chemical, biochemical, or biological substance or structure, present in or at a system of interest, such as a container, a reservoir, a reactor, a tube, a line, a vessel, a lumen, a tissue, an organ, or an organism, wherein a fluid or another viscoelastic medium or material comprises the analyte of interest, by measuring the concentration of the analyte of interest in a measurement chamber, wherein the measurement chamber comprises an effective number of binding sites (*N_{b}*), wherein the binding sites have a binding affinity to the analyte of interest, wherein the measurement chamber has an effective volume (*V_{ch}*) in which the analyte of interest has a significant probability to encounter the binding sites, and wherein the method comprises the step of providing a time-dependent sampling of the analyte of interest, by providing a time-dependent exchange of analyte between the system of interest and the effective volume (*V_{ch}*) of the measurement chamber, by performing at least one exchange modulation cycle comprising the following successive steps:
a) facilitating a primary exchange phase having a characteristic time of primary exchange (τ*_{pr.exch.}*) and a duration of primary exchange (*t_{pr.exch.}*)*;*
b) facilitating a primary-to-secondary switching phase having a characteristic primary-to-secondary switching time (*τ_{pr.sec.switch}*) and a primary-to-secondary switching duration (*t_{pr.sec.switch}*); and
c) facilitating a secondary exchange phase having a characteristic time of secondary exchange (τ*_{sec.exch}*.) and a duration of secondary exchange (*t_{sec.exch}.*)*,*
wherein the exchange modulation cycle is repeated for any time-dependent sampling further provided,
**characterised in that**
- the number of binding sites (*N_{b}*) and/or the effective volume (*V_{ch}*) of the measurement chamber is selected such that the effective volumetric binding site concentration (*C_{b,ch}*) in the measurement chamber is present in excess compared to the effective equilibrium dissociation constant (*K_{d}*) of the affinity binding between analyte of interest and binding sites, where *C_{b,ch}* is expressed as *N_{b}*/*V_{ch}*;
- the sum of the duration of primary exchange (*t_{pr.exch.}*) and the primary-to-secondary switching duration (*t_{pr.sec.switch}*) and the duration of secondary exchange (*t_{sec.exch.}*) is larger than a characteristic time-to-equilibrium (*τ*) in the measurement chamber;
- the concentration of the analyte of interest is determined by direct or indirect measuring the time-development of the amount of analyte of interest bound to at least one or more binding sites; and
- the direct or indirect measuring of the time-development of the amount of analyte of interest bound to at least one or more binding sites involves at least two measurements performed at different time-points in at least one exchange modulation cycle.

2. Method according to claim 1, wherein the binding sites are present on or in a supporting structure, such as a planar surface, a surface with concave or convex structure, a chemically and/or physically patterned surface, a particle, a polymer, or a porous matrix, and/or wherein the binding sites are present in said fluid or another viscoelastic medium or material comprising the analyte of interest.

3. Method according to claim 1 or 2, wherein after step c), the exchange modulation cycle is repeated by performing the following step:
d) facilitating a secondary-to-primary switching phase having a characteristic secondary-to-primary switching time (*τ_{sec.pr.switch}*) and a secondary-to-primary switching duration (*t_{sec.pr.switch}*), and wherein:
- the sum of the duration of primary exchange (*t_{pr.exch.}*) and the primary-to-secondary switching duration (*t_{pr.sec.switch}*) and the duration of secondary exchange (*t_{sec.exch.}*) and the duration of secondary-to-primary switching duration (*t_{sec.pr.switch}*) is larger than a characteristic time-to-equilibrium (*τ*) in the measurement chamber.

4. Method according to any of the preceding claims, wherein the time-dependent sampling of the analyte of interest is effectuated by time-dependent exchange of analyte by diffusion, advection, or by another active or passive physicochemical analyte transport method, or by a combination thereof.

5. Method according to any of the preceding claims, wherein the duration of primary exchange (*t_{pr.exch}*.)*:*
- is smaller than the characteristic time-to-equilibrium (*τ*) in the measurement chamber;
- is larger than the characteristic time of primary exchange (*τ_{pr.exch.}*); or
- is larger than one-hundredth of the characteristic time of primary exchange (τ*_{pr.exch.}*).

6. Method according to any of the preceding claims, wherein:
- the primary-to-secondary switching duration (*t_{pr.sec.switch}*) is larger than the characteristic primary-to-secondary switching time (τ*_{pr.sec.switch}*), and/or the characteristic primary-to-secondary switching time (τ*_{pr.sec.switch}*) is smaller than the characteristic time-to-equilibrium (*τ*) in the measurement chamber; and/or
- in case of step d), the secondary-to-primary switching duration (*t_{sec.pr.switch}*) is larger than the characteristic secondary-to-primary switching time (τ*_{sec.pr.switch}*)*,* and/or the characteristic secondary-to-primary switching time (τ*_{sec.pr.switch}*) is smaller than the characteristic time-to-equilibrium (*τ*) in the measurement chamber.

7. Method according to any of the preceding claims, wherein the sum of the duration of primary exchange (*t_{pr.exch.}*) and the primary-to-secondary switching duration (*t_{pr.sec.switch}*) is smaller than the characteristic time-to-equilibrium (*τ*) in the measurement chamber.

8. Method according to any of the preceding claims, wherein the duration of the secondary exchange (*t_{sec.exch.}*) is smaller than the characteristic time of secondary exchange (τ_{*sec.exch*.}).

9. Method according to any of the preceding claims, wherein the at least one exchange modulation cycle comprises two or more exchange modulation cycles and, optionally, wherein the measuring of the time-development of the amount of analyte of interest bound to at least one or more binding sites involves at least two measurements performed at different time-points in at least one exchange modulation cycle.

10. Method according to any of the preceding claims, wherein the facilitating of the phases during the at least one exchange modulation cycle is performed by diffusion, advection, or by another active or passive physicochemical analyte transport method, or by a combination thereof.

11. Method according to any of the preceding claims, wherein the phases of the at least one exchange modulation cycle are effectuated by controlling the transport method in time.

12. Method according to any of the preceding claims, wherein the increase or decrease of the time-development of the amount of analyte of interest bound to at least one or more binding sites during an exchange modulation cycle depends on the amount of analyte of interest bound to at least one or more binding sites in said exchange modulation cycle and in a previous exchange modulation cycle.

13. Method according to any of the preceding claims, wherein the binding of the analyte of interest to a binding site is measured by:
- a property of the analyte of interest, such as by charge, refractive index, fluorescence, luminescence, absorption, change of conformation, enzymatic activity, colour, or mass; or
- a signal from another object, such as a molecule, substance, particle, label, surface, or a combination thereof, for example by energy transfer, resonance, scattering, absorption, motion, charge, refractive index, fluorescence, luminescence, change of conformation, enzymatic activity, colour, or mass,
wherein the measurement involves binding, conversion, competition, inhibition, displacement, amplification, molecular cascade or sandwich formation, or a combination thereof.

14. System for continuously monitoring or intermittently testing at least one analyte of interest, wherein the system comprises:
- a measurement chamber comprising a number of binding sites (*N_{b}*)*,* wherein the binding sites are able to bind the analyte of interest, and wherein the measurement chamber has an effective volume (*V_{ch}*);
- at least one exchange port, such as a tube, a channel, an opening, a connector, a valve, a permeable or semipermeable material, or a membrane, for time-dependent sampling of the analyte of interest involving transport into and/or out of the measurement chamber,
wherein the system is configured to perform the method according to any of the preceding claims.

15. System according to claim 14, wherein the system is configured to monitor:
- one analyte of interest; or
- multiple analytes of interest, wherein the measurement chamber comprises multiple binding sites, and wherein each of the multiple binding sites is able to bind a specific analyte of interest selected from the group of multiple analytes of interest to be monitored, and, optionally, wherein the system is further configured to perform multiple methods according to any of claims 1-13 in parallel, wherein each of the methods performed monitors one analyte of interest of the multiple of analytes of interest to be monitored.

## Patentansprüche

1. Verfahren zum kontinuierlichen Überwachen oder intermittierenden Untersuchen eines bestimmten Analyten, wie etwa einem chemischen, biochemischen oder biologischen Stoff oder einer solchen Struktur, der/die in oder an einem bestimmten System, wie etwa einem Behälter, einem Reservoir, einem Reaktor, einem Rohr, einer Leitung, einem Gefäß, einem Lumen, einem Gewebe, einem Organ oder einem Organismus vorhanden ist, wobei ein Fluid oder ein anderes viskoelastisches Medium oder Material den bestimmten Analyten umfasst, durch Messen der Konzentration des bestimmten Analyten in einer Messkammer, wobei die Messkammer eine effektive Anzahl von Bindungsstellen *(N_{b})* umfasst*,* wobei die Bindungsstellen eine Bindungsaffinität zu dem bestimmten Analyten aufweisen, wobei die Messkammer ein effektives Volumen *(V_{ch})* aufweist, bei dem der bestimmte Analyt eine signifikante Wahrscheinlichkeit aufweist, auf die Bindungsstellen zu treffen, und wobei das Verfahren den Schritt des Bereitstellens einer zeitabhängigen Probenahme des bestimmten Analyten umfasst, indem ein zeitabhängiger Austausch von Analyt zwischen dem bestimmten System und dem effektiven Volumen *(V_{ch})* der Messkammer bereitgestellt wird, indem mindestens ein Austauschmodulationszyklus durchgeführt wird, der folgende aufeinanderfolgenden Schritte umfasst:
a) Ermöglichen einer Primäraustauschphase mit einer charakteristischen Zeit des Primäraustausches *(τ_{pr.exch})* und einer Dauer des Primäraustausches *(t_{pr.exch})*,
b) Ermöglichen einer Primär-Sekundär-Umschaltphase mit einer charakteristischen Primär-Sekundär-Umschaltzeit *(τ_{pr.sec.switch})* und einer Primär-Sekundär-Umschaltdauer *(tpr.sec.switch.)*; und
c) Ermöglichen einer Sekundäraustauschphase mit einer charakteristischen Zeit des Sekundäraustausches *(τ_{sec.exch.})* und einer Dauer des Sekundäraustausches *(t_{sec.exch.})*,
wobei der Austauschmodulationszyklus für jede weiter vorgesehene zeitabhängige Probenahme wiederholt wird,
**dadurch gekennzeichnet, dass**
- die Anzahl der Bindungsstellen *(N_{b})* und/oder das effektive Volumen *(V_{ch})* der Messkammer so gewählt ist, dass die effektive volumetrische Bindungsstellenkonzentration *(C_{b,ch})* in der Messkammer im Vergleich zur effektiven Gleichgewichtsdissoziationskonstante *(K_{d})* der Affinitätsbindung zwischen bestimmtem Analyten und Bindungsstellen im Überschuss vorhanden ist, wobei *C_{b,ch}* ausgedrückt wird als *N_{b}*/*V_{ch}*;
- die Summe aus der Dauer des Primäraustausches *(t_{pr.exch.})* und der Primär-Sekundär-Umschaltdauer *(t_{pr.sec.switch})* und der Dauer des Sekundäraustausches *(t_{sec.exch.})* größer ist als eine charakteristische Zeit bis zum Gleichgewicht (τ) in der Messkammer;
- die Konzentration des bestimmten Analyten durch direktes oder indirektes Messen der Zeitentwicklung der Menge des bestimmten Analyten, die an mindestens eine oder mehrere Bindungsstellen gebunden ist, bestimmt wird; und
- das direkte oder indirekte Messen der Zeitentwicklung der Menge an bestimmtem Analyten, die an mindestens eine oder mehrere Bindungsstellen gebunden ist, mindestens zwei Messungen einbezieht, die zu unterschiedlichen Zeitpunkten in mindestens einem Austauschmodulationszyklus durchgeführt werden.

2. Verfahren gemäß Anspruch 1, wobei die Bindungsstellen auf oder in einer Trägerstruktur, wie etwa einer planaren Oberfläche, einer Oberfläche mit konkaver oder konvexer Struktur, einer chemisch und/oder physikalisch gemusterten Oberfläche, einem Partikel, einem Polymer oder einer porösen Matrix, vorhanden sind, und/oder wobei die Bindungsstellen in dem Fluid oder einem anderen viskoelastischen Medium oder Material vorhanden sind, das den bestimmten Analyten umfasst.

3. Verfahren gemäß Anspruch 1 oder 2, wobei nach Schritt c) der Austauschmodulationszyklus durch Durchführen des folgenden Schritts wiederholt wird:
d) Ermöglichen einer Sekundär-Primär-Umschaltphase mit einer charakteristischen Sekundär-Primär-Umschaltzeit *(τ_{sec.pr.switch})* und einer Sekundär-Primär-Umschaltdauer *(t_{sec.pr.switch}),* und wobei:
- die Summe aus der Dauer des Primäraustausches *(t_{pr.exch.})* und der Primär-Sekundär-Umschaltdauer *(t_{pr.sec.switch})* und der Dauer des Sekundäraustausches *(t_{sec.exch.})* und der Dauer der Sekundär-Primär-Umschaltdauer *(t_{sec.pr.switch})* größer ist als eine charakteristische Zeit bis zum Gleichgewicht (τ) in der Messkammer.

4. Verfahren gemäß einem der vorhergehenden Ansprüche, wobei die zeitabhängige Probenahme des bestimmten Analyten durch zeitabhängigen Austausch des Analyten durch Diffusion, Advektion oder durch ein anderes aktives oder passives physikochemisches Analyt-Transportverfahren oder durch eine Kombination davon bewirkt wird.

5. Verfahren gemäß einem der vorhergehenden Ansprüche, wobei die Dauer des Primäraustausches *(t_{pr.exch.})*:
- kleiner als die charakteristische Zeit bis zum Gleichgewicht (τ) in der Messkammer ist;
- größer als die charakteristische Zeit des Primäraustausches *(τ_{pr.exch.})* ist; oder
- größer als ein Hundertstel der charakteristischen Zeit des Primäraustausches *(τ_{pr.exch.})* ist.

6. Verfahren gemäß einem der vorhergehenden Ansprüche, wobei:
- die Primär-Sekundär-Umschaltdauer *(t_{pr.sec.switch})* größer ist als die charakteristische Primär-Sekundär-Umschaltzeit *(τ_{pr.sec.switch}),* und/oder die charakteristische Primär-Sekundär-Umschaltzeit *(τ_{pr.sec.switch})* kleiner ist als die charakteristische Zeit bis zum Gleichgewicht (*τ*) in der Messkammer; und/oder
- im Fall von Schritt d) die Sekundär-Primär-Umschaltdauer *(t_{sec.pr.switch})* größer ist als die charakteristische Sekundär-Primär-Umschaltzeit *(τ_{sec.pr.switch}),* und/oder die charakteristische Sekundär-Primär-Umschaltzeit *(τ_{sec.pr.switch})* kleiner ist als die charakteristische Zeit bis zum Gleichgewicht (*τ*) in der Messkammer.

7. Verfahren gemäß einem der vorhergehenden Ansprüche, wobei die Summe aus der Dauer des Primäraustausches *(t_{pr.exch.})* und der Primär-Sekundär-Umschaltdauer *(t_{pr.sec.switch})* kleiner ist als die charakteristische Zeit bis zum Gleichgewicht (*τ*) in der Messkammer.

8. Verfahren gemäß einem der vorhergehenden Ansprüche, wobei die Dauer des Sekundäraustausches *(t_{sec.exch.})* kleiner ist als die charakteristische Zeit des Sekundäraustausches *(τsec.exch.).*

9. Verfahren gemäß einem der vorhergehenden Ansprüche, wobei der mindestens eine Austauschmodulationszyklus zwei oder mehr Austauschmodulationszyklen umfasst und wobei optional das Messen der Zeitentwicklung der Menge des bestimmten Analyten, der an mindestens eine oder mehrere Bindungsstellen gebunden ist, mindestens zwei Messungen einbezieht, die zu unterschiedlichen Zeitpunkten in mindestens einem Austauschmodulationszyklus durchgeführt werden.

10. Verfahren gemäß einem der vorhergehenden Ansprüche, wobei das Ermöglichen der Phasen während des mindestens einen Austauschmodulationszyklus durch Diffusion, Advektion oder durch ein anderes aktives oder passives physikochemisches Analyt-Transportverfahren oder durch eine Kombination davon durchgeführt wird.

11. Verfahren gemäß einem der vorhergehenden Ansprüche, wobei die Phasen des mindestens einen Austauschmodulationszyklus durch zeitliches Steuern des Transportverfahrens bewirkt werden.

12. Verfahren gemäß einem der vorhergehenden Ansprüche, wobei die Zunahme oder Abnahme der Zeitentwicklung der Menge des bestimmten Analyten, der an mindestens eine oder mehrere Bindungsstellen während eines Austauschmodulationszyklus gebunden ist, von der Menge des bestimmten Analyten abhängt, der an mindestens eine oder mehrere Bindungsstellen in dem Austauschmodulationszyklus und in einem vorherigen Austauschmodulationszyklus gebunden ist.

13. Verfahren gemäß einem der vorhergehenden Ansprüche, wobei die Bindung des bestimmten Analyten an eine Bindungsstelle gemessen wird durch:
- eine Eigenschaft des bestimmten Analyten, wie etwa durch Ladung, Brechungskoeffizient, Fluoreszenz, Lumineszenz, Absorption, Konformationsänderung, enzymatische Aktivität, Farbe oder Masse; oder
- ein Signal von einem anderen Objekt, wie etwa einem Molekül, Stoff, Partikel, Etikett, einer Oberfläche oder einer Kombination davon, zum Beispiel durch Energieübertragung, Resonanz, Streuung, Absorption, Bewegung, Ladung, Brechungskoeffizient, Fluoreszenz, Lumineszenz, Konformationsveränderung, enzymatische Aktivität, Farbe oder Masse,
wobei die Messung Bindung, Umwandlung, Konkurrenz, Hemmung, Verdrängung, Amplifikation, Molekülkaskade oder Sandwichbildung oder eine Kombination davon einbezieht.

14. System zum kontinuierlichen Überwachen oder intermittierenden Untersuchen mindestens eines bestimmten Analyten, wobei das System umfasst:
- eine Messkammer, umfassend eine Anzahl von Bindungsstellen *(N_{b}),* wobei die Bindungsstellen in der Lage sind, den bestimmten Analyten zu binden, und wobei die Messkammer ein effektives Volumen *(V_{ch})* aufweist;
- mindestens einen Austauschanschluss, wie etwa ein Rohr, einen Kanal, eine Öffnung, einen Anschluss, ein Ventil, ein permeables oder semipermeables Material oder eine Membran, zur zeitabhängigen Probenahme des bestimmten Analyten unter Einbeziehung des Transports in die und/oder aus der Messkammer,
wobei das System so konfiguriert ist, dass es dass Verfahren gemäß einem der vorhergehenden Ansprüche durchführt.

15. System gemäß Anspruch 14, wobei das System so konfiguriert ist, dass es Folgendes überwacht:
- einen bestimmten Analyten; oder
- mehrere bestimmte Analyten, wobei die Messkammer mehrere Bindungsstellen umfasst und wobei jede der mehreren Bindungsstellen in der Lage ist, einen spezifischen bestimmten Analyten zu binden, der ausgewählt ist aus der Gruppe von mehreren zu überwachenden bestimmten Analyten, und wobei das System optional ferner so konfiguriert ist, dass es mehrere Verfahren gemäß einem der Ansprüche 1-13 parallel durchführt, wobei jedes der durchgeführten Verfahren einen bestimmten Analyten der mehreren zu überwachenden bestimmten Analyten überwacht.

## Revendications

1. Procédé pour la surveillance continue ou le test intermittent d'un analyte d'intérêt, tel qu'une substance ou une structure chimique, biochimique ou biologique, présent dans ou au niveau d'un système d'intérêt, tel qu'un récipient, un réservoir, un réacteur, un tube, une conduite, un vaisseau, une lumière, un tissu, un organe ou un organisme, dans lequel un fluide ou un autre milieu ou matériau viscoélastique comprend l'analyte d'intérêt, en mesurant la concentration de l'analyte d'intérêt dans une chambre de mesure, dans lequel la chambre de mesure comprend un nombre effectif de sites de liaison *(N_{b}),* dans lequel les sites de liaison ont une affinité de liaison avec l'analyte d'intérêt, dans lequel la chambre de mesure a un volume effectif *(V_{ch})* dans lequel l'analyte d'intérêt a une probabilité significative de rencontrer les sites de liaison, et dans lequel le procédé comprend l'étape de fourniture d'un échantillonnage dépendant du temps de l'analyte d'intérêt, en assurant un échange dépendant du temps de l'analyte entre le système d'intérêt et le volume effectif *(V_{ch})* de la chambre de mesure, en effectuant au moins un cycle de modulation d'échange comprenant les étapes successives suivantes :
a) réalisation d'une phase d'échange primaire ayant une constante de temps d'échange primaire *(τ_{pr.exch})* et une durée d'échange primaire *(t_{pr.exch}) ;*
b) réalisation d'une phase de commutation entre l'échange primaire et l'échange secondaire ayant une constante de temps de commutation primaire-secondaire *(τ_{pr.sec.switch})* et une durée de commutation primaire-secondaire *(t_{pr.sec.switch})* ; et
c) réalisation d'une phase d'échange secondaire ayant une constante de temps d'échange secondaire *(τ_{sec.exch})* et une durée d'échange secondaire *(t_{sec.exch.})*,
dans lequel le cycle de modulation d'échange est répété pour tout échantillonnage dépendant du temps ultérieurement réalisé,
**caractérisé en ce que**
- le nombre de sites de liaison *(N_{b})* et/ou le volume effectif *(V_{ch})* de la chambre de mesure est choisi de telle sorte que la concentration volumétrique effective en sites de liaison *(C_{b,ch})* dans la chambre de mesure soit présente en excès par rapport à la constante de dissociation à l'équilibre effective *(K_{d})* de la liaison d'affinité entre l'analyte d'intérêt et les sites de liaison, où *C_{b,ch}* est exprimée par *N_{b}*/*V_{ch}* ;
- la somme de la durée de l'échange primaire *(t_{pr.exch.})* et de la durée de commutation entre l'échange primaire et l'échange secondaire *(t_{pr.sec.switch})* et de la durée de l'échange secondaire *(t_{sec.exch.})* est supérieure à une constante de temps d'établissement d'équilibre (τ) dans la chambre de mesure ;
- la concentration de l'analyte d'intérêt est déterminée par une mesure directe ou indirecte de l'évolution dans le temps de la quantité d'analyte d'intérêt liée à au moins un ou plusieurs sites de liaison ; et
- la mesure directe ou indirecte de l'évolution dans le temps de la quantité d'analyte d'intérêt liée à au moins un ou plusieurs sites de liaison implique au moins deux mesures effectuées à des moments différents dans au moins un cycle de modulation d'échange.

2. Procédé selon la revendication 1, dans lequel les sites de liaison sont présents sur ou dans une structure de support, telle qu'une surface plane, une surface avec une structure concave ou convexe, une surface à motifs chimiques et/ou physiques, une particule, un polymère ou une matrice poreuse, et/ou dans lequel les sites de liaison sont présents dans ledit fluide ou un autre milieu ou matériau viscoélastique comprenant l'analyte d'intérêt.

3. Procédé selon la revendication 1 ou 2, dans lequel après l'étape c), le cycle de modulation d'échange est répété en effectuant l'étape suivante :
d) réalisation d'une phase de commutation entre l'échange secondaire et l'échange primaire ayant une constante de temps de commutation secondaire-primaire *(τ_{sec.pr.switch})* et une durée de commutation secondaire-primaire *(t_{sec.pr.switch}),* et dans lequel :
- la somme de la durée de l'échange primaire *(t_{pr.exch.})* et de la durée de commutation primaire-secondaire *(t_{pr.sec.switch})* et de la durée de l'échange secondaire *(t_{sec.exch.})* et de la durée de commutation secondaire-primaire *(t_{sec.pr.switch})* est supérieure à une caractéristique temps-équilibre (τ) dans la chambre de mesure.

4. Procédé selon l'une quelconque des revendications précédentes, dans lequel l'échantillonnage dépendant du temps de l'analyte d'intérêt est effectué par échange dépendant du temps de l'analyte par diffusion, advection, ou par un autre procédé de transport d'analyte physico-chimique actif ou passif, ou par une combinaison de ceux-ci.

5. Procédé selon l'une quelconque des revendications précédentes, dans lequel la durée de l'échange primaire *(t_{pr.exch.})* :
- est inférieure à la constante temps-équilibre (τ) dans la chambre de mesure ;
- est supérieure à la constante d'échange primaire *(τ_{pr.exch..}),* ou
- est supérieure au centième de la constante d'échange primaire *(τ_{pr.exch.}).*

6. Procédé selon l'une quelconque des revendications précédentes, dans lequel :
- la durée de commutation entre l'échange primaire et l'échange secondaire *(t_{pr.sec.switch})* est supérieure à la constante de temps de commutation primaire-secondaire *(τ_{pr.sec.switch}),* et/ou la constante de temps de commutation primaire-secondaire *(τ_{pr.sec.switch})* est inférieure à la constante de temps d'établissement d'équilibre (*τ*) dans la chambre de mesure ; et/ou
- dans le cas de l'étape d), la durée de commutation entre l'échange secondaire et l'échange primaire *(t_{sec.pr.switch})* est supérieure à la constante de temps de commutation secondaire-primaire *(τ_{sec.pr.switch}),* et/ou la constante de temps de commutation secondaire-primaire *(τ_{sec.pr.switch})* est inférieure à la constante de temps d'établissement d'équilibre (*τ*) dans la chambre de mesure.

7. Procédé selon l'une quelconque des revendications précédentes, dans lequel la somme de la durée de l'échange primaire *(t_{pr.exch.})* et de la durée de commutation primaire-secondaire *(t_{pr.sec.switch})* est inférieure à la constante de temps d'établissement d'équilibre (*τ*) dans la chambre de mesure.

8. Procédé selon l'une quelconque des revendications précédentes, dans lequel la durée de l'échange secondaire *(t_{sec.exch.})* est inférieure à la constante de temps de l'échange secondaire *(τsec.exch.).*

9. Procédé selon l'une quelconque des revendications précédentes, dans lequel l'au moins un cycle de modulation d'échange comprend deux cycles de modulation d'échange ou plus et, facultativement, dans lequel la mesure de l'évolution dans le temps de la quantité d'analyte d'intérêt liée à au moins un ou plusieurs sites de liaison implique au moins deux mesures effectuées à des moments différents dans au moins un cycle de modulation d'échange.

10. Procédé selon l'une quelconque des revendications précédentes, dans lequel la réalisation des phases pendant l'au moins un cycle de modulation d'échange est effectuée par diffusion, advection, ou par un autre procédé de transport d'analyte physico-chimique actif ou passif, ou par une combinaison de ceux-ci.

11. Procédé selon l'une quelconque des revendications précédentes, dans lequel les phases de l'au moins un cycle de modulation d'échange sont effectuées en commandant le procédé de transport dans le temps.

12. Procédé selon l'une quelconque des revendications précédentes, dans lequel l'augmentation ou la diminution de l'évolution dans le temps de la quantité d'analyte d'intérêt liée à au moins un ou plusieurs sites de liaison au cours d'un cycle de modulation d'échange dépend de la quantité d'analyte d'intérêt liée à au moins un ou plusieurs sites de liaison dans ledit cycle de modulation d'échange et dans un cycle de modulation d'échange précédent.

13. Procédé selon l'une quelconque des revendications précédentes, dans lequel la liaison de l'analyte d'intérêt à un site de liaison est mesurée par :
- une propriété de l'analyte d'intérêt, telle que par charge, indice de réfraction, fluorescence, luminescence, absorption, changement de conformation, activité enzymatique, couleur ou masse ; ou
- un signal provenant d'un autre objet, tel qu'une molécule, une substance, une particule, un marqueur, une surface, ou une combinaison de ceux-ci, par exemple par transfert d'énergie, résonance, diffusion, absorption, mouvement, charge, indice de réfraction, fluorescence, luminescence, changement de conformation, activité enzymatique, couleur, ou masse,
dans lequel la mesure implique une liaison, une conversion, une compétition, une inhibition, un déplacement, une amplification, une cascade moléculaire ou une formation de sandwich, ou une combinaison de ceux-ci.

14. Système pour la surveillance continue ou le test intermittent d'au moins un analyte d'intérêt, dans lequel le système comprend :
- une chambre de mesure comprenant un certain nombre de sites de liaison *(N_{b}),* dans lequel les sites de liaison sont capables de se lier à l'analyte d'intérêt, et dans lequel la chambre de mesure a un volume effectif *(V_{ch})* ;
- au moins un port d'échange, tel qu'un tube, un canal, une ouverture, un connecteur, une vanne, un matériau perméable ou semi-perméable, ou une membrane, pour le prélèvement en fonction du temps de l'analyte d'intérêt impliquant le transport dans et/ou hors de la chambre de mesure,
dans lequel le système est conçu pour exécuter le procédé selon l'une quelconque des revendications précédentes.

15. Système selon la revendication 14, dans lequel le système est configuré pour surveiller :
- un analyte d'intérêt ; ou
- de multiples analytes d'intérêt, dans lequel la chambre de mesure comprend de multiples sites de liaison, et dans lequel chacun des multiples sites de liaison est capable de se lier à un analyte d'intérêt spécifique choisi dans le groupe de multiples analytes d'intérêt à surveiller, et, facultativement, dans lequel le système est en outre configuré pour réaliser de multiples procédés selon l'une quelconque des revendications 1 à 13 en parallèle, dans lequel chacun des procédés réalisés surveille un analyte d'intérêt parmi les multiples analytes d'intérêt à surveiller.
